# EUROPEAN PATENT APPLICATION

(11) **EP 2 191 768 A1**
(43) Date of publication of application: **02.06.2010**
(21) Application number: 08829847.6
(22) Date of filing: 28.08.2008
(51) Int. Cl.: A61B 1/00, A61B 5/06, A61B 5/07, G01B 7/00

(54) **POSITION SENSOR, MEDICAL DEVICE GUIDING SYSTEM, POSITION SENSING METHOD, AND MEDICAL DEVICE GUIDING METHOD**

(30) Priority: 07.09.2007 JP 2007233321
(71) Applicant: Olympus Medical Systems Corp., Tokyo 151-0072 (JP)
(72) Inventor: CHIBA, Atsushi, Tokyo 151-0072 (JP); KIMURA, Atsushi, Tokyo 151-0072 (JP); SATO, Ryoji, Tokyo 151-0072 (JP); UCHIYAMA, Akio, Tokyo 151-0072 (JP); KAWANO, Hironao, Tokyo 151-0072 (JP); TAKAHASHI, Masaki, Tokyo 151-0072 (JP)
(74) Representative: von Hellfeld, Axel
(86) International application number: PCT/JP2008/065450
(87) International publication number: WO 2009/031456

(57) **Abstract**

It is an object of the present invention to stably perform a position detecting process of a detected object having an LC marker contained therein. A position detecting device (10) according to the present invention includes plural sets of drive coils (D_{X}, D_{Y}, D_{Z}), a magnetic-field-generating-coil switching unit (15) that switches drive coils that apply a magnetic field to an LC marker (2a) in a capsule endoscope (2) among the sets of the drive coils (D_{X}, D_{Y}, D_{Z}), a storage unit (19) that stores predetermined information of a selecting condition of the drive coils, and a control unit (20), thereby detecting a position of the capsule endoscope (2) based on a detection result of an induced magnetic field from the LC marker (2a). The control unit (20) determines a selecting condition of the drive coils based on the predetermined information, and selects drive coils that satisfy the determined selecting condition from the sets of the drive coils (D_{X}, D_{Y}, D_{Z}), thereby controlling the magnetic-field-generating-coil switching unit (15) to switch to the selected drive coils.

## Description

### TECHNICAL FIELD

The present invention relates to a position detecting device, a medical device guidance system, a position detecting method, and a medical device guiding method in which a magnetic field is applied to a resonance circuit between a coil and a capacitor (hereinafter, "LC marker") contained in a detected object so as to generate an induced magnetic field in the LC marker, and the induced magnetic field is detected to detect a position of the detected object.

### BACKGROUND ART

Conventionally, a capsule medical device that can be introduced into a digestive canal of a subject such as a patient has been put to practical use. The capsule medical device is swallowed from the mouth of the subject to acquire in-vivo information such as in-vivo images in the subject, while moving in the digestive canal with peristaltic movements, and wirelessly transmits acquired in-vivo information to a receiving device located outside the subject. The capsule medical device sequentially acquires in-vivo information of the subject, since introduction into the digestive canal of the subject until it is naturally discharged from the subject.

Further, there has been an LC marker-type position detecting device that has an LC marker contained therein in the capsule medical device to detect the position of the capsule medical device inside the subject by using the LC marker. Specifically, the LC marker-type position detecting device includes a magnetic-field generation coil (hereinafter, "drive coil") that generates a magnetic field and a detection coil (hereinafter, "sense coil") that detects a magnetic field, applies the magnetic field generated by the drive coil to the LC marker in the capsule medical device, thereby detecting an induced magnetic field generated from the LC marker by the sense coil, and detects the position of the capsule medical device in the subject based on the detected induced magnetic field.

Such position detecting devices include a type of position detecting device that includes a plurality of drive coils for generating different magnetic fields, and switches the drive coil for applying the magnetic field to an LC marker in a capsule medical device according to the detected position thereof (see, for example, Patent Document 1), and another type of position detecting device that includes a plurality of sense coils and detects the position of a capsule medical device by selectively using the sense coil having a higher detection value of an induced magnetic field generated from an LC marker in the capsule medical device (see, for example, Patent Document 2).

Patent Document 1: Japanese Laid-open Patent Publication No. 2007-175317
Patent Document 2: Japanese Laid-open Patent Publication No. 2006-271520

### DISCLOSURE OF INVENTION

### PROBLEM TO BE SOLVED BY THE INVENTION

However, in the conventional position detecting devices mentioned above, every time a detected object exemplified by a capsule medical device having an LC marker contained therein is displaced, an operation in which the drive coil is repeatedly switched in a short time according to the position of the detected object has to be performed frequently, thereby making the transient characteristic and the temperature characteristic after switching of the drive coil unstable. Further, an unstable magnetic field generated by the drive coil is applied to the LC marker, and as a result, a position detecting process of the detected object also becomes unstable.

Because simply selecting a sense coil having a higher detection value of the induced magnetic field from the LC marker, a sense coil in which a detection value of the magnetic field is saturated due to being positioned near the drive coil that has generated the magnetic field can be selected. Accordingly, reliability of the detection value of the induced magnetic field from the LC marker deteriorates, and as a result, the position detecting process of the detected object becomes unstable.

The present invention has been achieved in view of the above problems, an object of the present invention is to provide a position detecting device, a medical device guidance system, a position detecting method, and a medical device guiding method that can stably perform a position detecting process of a detected object having an LC marker contained therein.

### MEANS FOR SOLVING PROBLEM

To solve the problems and achieve the object, a position detecting device according to the present invention includes a detected object that includes a circuit with at least one internal coil; a first magnetic-field generator that includes at least one magnetic-field generating coil for generating a first magnetic field to a detection space of the detected object; a plurality of detection coils that detect an induced magnetic field generated from the internal coil cause by the first magnetic field; a magnetic-field-generating-coil switching unit that selects at least one magnetic-field generating coil to be operated from the magnetic-field generating coils in the first magnetic-field generator; a storage unit that stores predetermined information for selecting at least one magnetic-field generating coil to be operated from the magnetic-field generating coils in the first magnetic-field generator; and a control unit that controls the magnetic-field-generating-coil switching unit to select the at least one magnetic-field generating coil to be operated among the magnetic-field generating coils in the first magnetic-field generator, based on at least one of a position and a direction of the detected object and based on the predetermined information.

In the position detecting device according to the present invention, the control unit hysteretically selects the magnetic-field generating coil based on the predetermined information.

In the position detecting device according to the present invention, the predetermined information is a continuous operation time of the selected magnetic-field generating coil.

In the position detecting device according to the present invention, the predetermined information is a selection sequence of the magnetic-field generating coil.

In the position detecting device according to the present invention, the predetermined information is information of the first magnetic field generated by the magnetic-field generating coils.

The position detecting device according to the present invention further includes at least one signal generator that outputs an alternating-current signal to the at least one magnetic-field generating coil. At a time of selecting the magnetic-field generating coil, the control unit stops an output of the signal generator in an active state, and after a predetermined time has passed, disconnects between the magnetic-field generating coil which is in an active state and the magnetic-field-generating-coil switching unit, then after a predetermined time has passed, connects the selected magnetic-field generating coil with the magnetic-field-generating-coil switching unit, and then after a predetermined time has passed, starts an output of the selected signal generator.

In the position detecting device according to the present invention, the magnetic-field generating coil which is in an active state is the selected magnetic-field generating coil.

The position detecting device according to the present invention further includes at least one signal generator that outputs an alternating-current signal to the at least one magnetic-field generating coil. At a time of selecting the magnetic-field generating coil, the control unit connects the selected magnetic-field generating coil with the magnetic-field-generating-coil switching unit, and after a predetermined time has passed, starts an output of the selected signal generator, then after a predetermined time has passed, stops an output of the signal generator, which has been in an active state, and then after a predetermined time has passed, disconnects between the magnetic-field generating coil, which has been in an active state, and the magnetic-field-generating-coil switching unit.

The position detecting device according to the present invention further includes an A/D converter that converts a detection result of the induced magnetic field detected by the detection coils to a digital signal; and an FFT calculator that calculates fast Fourier transform with respect to a detection result converted to the digital signal. The control unit stops an operation of at least one of the A/D converter and the FFT calculator during a period where the magnetic-field-generating-coil switching unit is selecting a magnetic-field generating coil.

The position detecting device according to the present invention further includes a position and direction calculator that calculates a position and a direction of the detected object based on detection results of the detection coils. The control unit stops calculation of a position and direction by the position and direction calculator during a period where the magnetic-field-generating-coil switching unit is selecting a magnetic-field generating coil.

In the position detecting device according to the present invention, the control unit selects an effective one among the detection coils according to the selected magnetic-field generating coil.

In the position detecting device according to the present invention, the detection coil further detects the first magnetic field, and the control unit selects an effective one among the detection coils according to a detection result of the first magnetic field detected by the detection coil.

A medical device guidance system according to the present invention includes a medical device that includes a circuit with at least one internal coil and a magnet, the medical device being a detected object; the position detecting device according to any one of the above inventions; and a second magnetic-field generator that generates a second magnetic field for guiding the medical device by operating the magnet.

A position detecting method according to the present invention includes a first magnetic-field generating step of generating a first magnetic field to a detection space of a detected object that includes a circuit with at least one internal coil; an induced magnetic-field detecting step of detecting, by a plurality of detection coils, an induced magnetic field generated from the internal coil caused by the first magnetic field; a position/direction calculating step of calculating at least one of a position and a direction of the detected object based on a detection result of the induced magnetic field; a magnetic-field generating-coil selecting step of selecting at least one magnetic-field generating coil for generating the first magnetic field, based on at least one of a position and a direction of the detected object and based on predetermined information for selecting at least one magnetic-field generating coil to be operated; and a control step of controlling a magnetic-field-generating-coil switching unit to switch to a magnetic-field generating coil selected at the magnetic-field generating-coil selecting step.

In the position detecting method according to the present invention, the magnetic-field generating-coil selecting step includes hysteretically selecting the magnetic-field generating coil based on the predetermined information.

The position detecting method according to the present invention further includes a signal-generator selecting step of selecting at least one signal generator that outputs an alternating-current signal to the at least one magnetic-field generating coil. The control step includes stopping an output of the signal generator in an active state that outputs an alternating-current signal to the magnetic-field generating coil in an active state; disconnecting, after a predetermined time has passed, between the magnetic-field generating coil which is in an active state and the magnetic-field-generating-coil switching unit; then connecting, after a predetermined time has passed, the magnetic-field generating coil selected at the magnetic-field generating-coil selecting step with the magnetic-field-generating-coil switching unit; and then causing, after a predetermined time has passed, the signal generator selected at the signal-generator selecting step to start an output of an alternating-current signal.

The position detecting method according to the present invention further includes a signal-generator selecting step of selecting at least one signal generator that outputs an alternating-current signal to the at least one magnetic-field generating coil. The control step includes connecting the magnetic-field generating coil selected at the magnetic-field generating-coil selecting step with the magnetic-field-generating-coil switching unit; starting, after a predetermined time has passed, an output of the signal generator selected at the signal-generator selecting step; then stopping, after a predetermined time has passed, an output of the signal generator which is in an active state; and then disconnecting, after a predetermined time has passed, between the magnetic-field generating coil which is in an active state and the magnetic-field-generating-coil switching unit.

The position detecting method according to the present invention further includes an A/D converting step of converting a detection result of the induced magnetic field detected by the detection coils to a digital signal by an A/D converter; and an FFT calculating step of performing fast Fourier transform with respect to a detection result converted to the digital signal by an FFT calculator. The control step includes stopping an operation of at least one of the A/D converter and the FFT calculator during a period where the magnetic-field-generating-coil switching unit is selecting the magnetic-field generating coil.

The position detecting method according to the present invention further includes a detection-coil selecting step of selecting an effective one among the detection coils according to the magnetic-field generating coil selected at the magnetic-field generating-coil selecting step.

A medical device guiding method according to the present invention includes a first magnetic-field generating step of generating a first magnetic field with respect to a detection space of a medical device which is a detected object, the medical device including a circuit with at least one internal coil; an induced magnetic-field detecting step of detecting an induced magnetic field generated from the internal coil caused by the first magnetic field by a plurality of detection coils; a position and direction calculating step of calculating at least one of a position and a direction of the detected object based on a detection result of the induced magnetic field; a magnetic-field generating-coil selecting step of selecting at least one magnetic-field generating coil for generating the first magnetic field, based on at least one of a position and a direction of the detected object and based on predetermined information for selecting at least one magnetic-field generating coil to be operated; a control step of controlling a magnetic-field-generating-coil switching unit to switch to a magnetic-field generating coil selected at the magnetic-field generating-coil selecting step; and a second magnetic-field generating step of generating a second magnetic field for guiding the medical device by operating the magnet.

### EFFECT OF THE INVENTION

According to the present invention, a selecting condition of the drive coil is determined in advance based on predetermined information stored in the storage unit, to select a drive coil that satisfies the determined selecting condition from a plurality of drive coils. A drive-coil switching operation is controlled so that the selected drive coil applies a magnetic field to a LC marker in a detected object, an induced magnetic field generated from the LC marker due to the applied magnetic field is detected by a plurality of sense coils, and position information of the detected object is detected based on the detection result of the field strength of the induced magnetic field acquired by the sense coils. Accordingly, an optimum drive coil can be selected from the drive coils according to the position and direction of the LC marker in a predetermined three-dimensional space, and an operation in which the drive coil in an active state is switched repeatedly in a short time according to the position of the detected object displaced in the three-dimensional space can be prevented. As a result, the transient characteristic and the temperature characteristic after switching of the drive coil can be stabilized, and thus it is possible to perform a position detecting process of a detected object having an LC marker contained therein in a stable manner.

### BRIEF DESCRIPTION OF DRAWINGS

FIG. 1 is a schematic block diagram of a configuration example of a capsule guidance system according to an embodiment of the present invention.
FIG. 2 is a schematic block diagram of a configuration example of a position detecting device according to the embodiment of the present invention.
FIG. 3 is a flowchart for exemplifying a process procedure performed by a drive coil selector that hysteretically selects drive coils in an active state.
FIG. 4 shows schematic diagrams for specifically explaining an operation of the drive coil selector that hysteretically selects drive coils in an active state.
FIG. 5 is a timing chart for exemplifying a switching control timing of a drive coil.
FIG. 6 is a flowchart for exemplifying a process procedure performed by a sense coil selector that selects a sense coil effective for a position information calculating process.
FIG. 7 is a schematic diagram for explaining an inappropriate sense coil in which a calibration value is saturated or substantially zero.
FIG. 8 is a schematic perspective view of a configuration example of a magnetic field generator including plural sets of drive coils.
FIG. 9 is a cross-sectional schematic diagram of a fitting part of drive coils of a longitudinal cross section of a cylindrical magnetic field generator.
FIG. 10 is a cross-sectional schematic diagram of a fitting part of drive coils of a vertical cross section of the cylindrical magnetic field generator.
FIG. 11 is a schematic diagram of a configuration example of a cylindrical member for fitting plural sets of drive coils.
FIG. 12 is a schematic diagram of a configuration example of a holding jig for holding a drive coil in a groove of the cylindrical member.
FIG. 13 is a schematic diagram of a configuration example of an L jig, which is a part of a holding jig.
FIG. 14 is a schematic diagram of a state for fitting the L jig to the cylindrical member.
FIG. 15 is a schematic diagram of a configuration example of an R jig, which is a part of a holding jig.
FIG. 16 is a flowchart for exemplifying an operation procedure for arranging and fixing a drive coil in a groove of the cylindrical member.
FIG. 17 is a schematic diagram of position adjustment of a holding jig with respect to a side of a drive coil.
FIG. 18 is a schematic diagram of position adjustment of the holding jig with respect to a corner of a drive coil.
FIG. 19 is a schematic diagram for explaining a procedure for holding down a drive coil in a groove by the holding jig.
FIG. 20 is a schematic diagram of a state that a curved portion of a drive coil is corrected to a linear shape by a correcting member.
FIG. 21 is a schematic block diagram of a modification of the position detecting device according to the embodiment of the present invention.
FIG. 22 is a timing chart of a modification of a switching control timing of a drive coil.
FIG. 23 is a cross-sectional schematic diagram of a modification of a groove for arranging and fixing a drive coil.

### EXPLANATIONS OF LETTERS OR NUMERALS

- 1: Capsule guidance system
- 2: Capsule endoscope
- 2a: LC marker
- 3: Magnetic-field generating device
- 4: Coil power source
- 5: Receiving antennas
- 6: Receiving unit
- 7: Input unit
- 8: Display unit
- 9: Storage unit
- 10: Position detecting device
- 11: Control device
- 12: Magnetic field generator
- 13a: to 13c Signal generator
- 14a: to 14c Amplifier
- 15: Magnetic-field-generating-coil switching unit
- 16a, 16b: Magnetic field detector
- 16c, 16c-1, 16c-2: Sense coil
- 17a: Filter
- 17b: Amplifier
- 17c: A/D converter
- 17d: FFT calculator
- 18: Position information calculator
- 19: Storage unit
- 19a: Magnetic-field information table
- 19b: Criteria information
- 20: Control unit
- 20a: Drive coil selector
- 17e, 20b: Sense coil selector
- 20c: Detection accuracy calculator
- 31: Cylindrical member
- 32a, 32b, 32c, 32d, 32e, 32f: Groove
- 32h: Undercut
- 33a, 33c-1, 33c-2, 33c-3, 33c-4: Wall
- 33c-5: Wire drawing part
- 34: Adhesive
- 35: Correcting member
- 41: Holding jig
- 42a, 42b: L jig
- 43a, 43b: R jig
- 44: Corner holding jig
- D_{X},: D_{Y}, and D_{Z} Drive coil
- S: Three-dimensional space

### BEST MODE(S) FOR CARRYING OUT THE INVENTION

Exemplary embodiments of a position detecting device, a medical device guidance system, a position detecting method, and a medical device guiding method according to the present invention will be explained below in detail with reference to the accompanying drawings. A position detecting device that detects the position of a capsule endoscope as a detected object by an LC marker method is explained below, by exemplifying a capsule guidance system that magnetically guides the capsule endoscope as one example of the capsule medical device by the magnetic field. However, the present invention is not limited by this embodiment.

### Embodiment

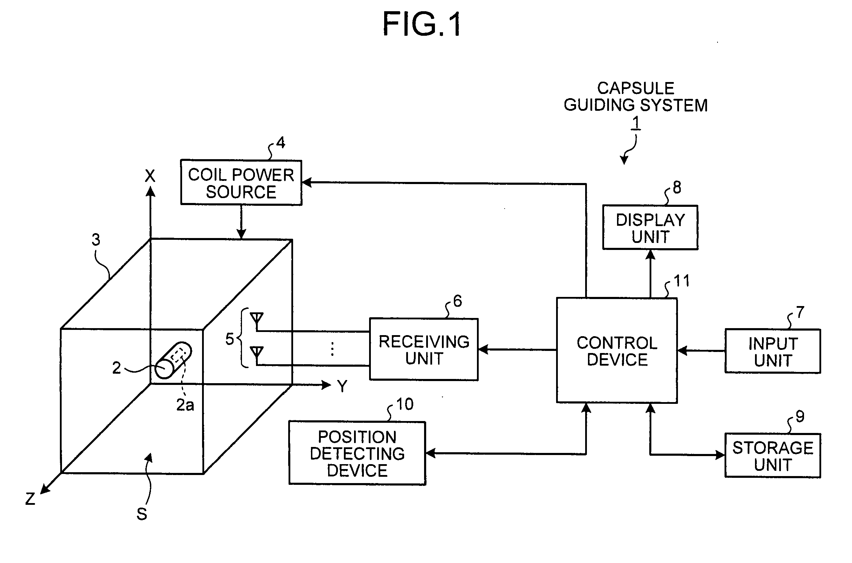

FIG. 1 is a schematic block diagram of a configuration example of the capsule guidance system according to an embodiment of the present invention. As shown in FIG. 1, a capsule guidance system 1 according to present the embodiment includes a capsule endoscope 2 that captures images inside a digestive canal of a subject such as a patient (hereinafter, "in-vivo images"), a magnetic-field generating device 3 that generates a magnetic field for magnetically guiding the capsule endoscope 2 introduced into the subject, and a coil power source 4 that supplies an electric current to a coil (an electromagnet) of the magnetic-field generating device 3. The capsule guidance system 1 includes a plurality of receiving antennas 5 arranged on a body surface of the subject, a receiving unit 6 that receives a radio signal from the capsule endoscope 2 via the receiving antennas 5, an input unit 7 that inputs various pieces of information, a display unit 8 that displays various pieces of information such as in-vivo images, a storage unit 9 that stores various pieces of information such as the in-vivo images, a position detecting device 10 that detects position information of the capsule endoscope 2 inside the subject according to the LC marker method, and a control device 11 that controls respective components in the capsule guidance system 1.

The capsule endoscope 2 is a capsule medical device that acquires in-vivo images of the subject (an example of in-vivo information), and has an imaging function and a wireless communication function. The capsule endoscope 2 is introduced into the digestive canal of the subject such as a patient (not shown), to sequentially capture the in-vivo images, while moving in the digestive canal of the subject. The capsule endoscope 2 then wirelessly transmits image signals including the in-vivo images of the subject sequentially to the receiving unit 6 outside the subject. The capsule endoscope 2 incorporates a magnetic body such as a permanent magnet or an electromagnet (hereinafter, "magnet") therein, and is guided while performing a desired operation by the magnetic field formed by the magnetic-field generating device 3. The capsule endoscope 2 also incorporates an LC marker 2a, which is an LC resonance circuit formed by using a coil and a capacitor. The LC marker 2a reacts to the magnetic field applied by the position detecting device 10 described later to generate an induced magnetic field, and emits the generated induced magnetic field to the outside of the capsule endoscope 2.

The magnetic-field generating device 3 is realized by combining a plurality of electromagnets such as a Helmholtz coil, to generate a magnetic field capable of guiding the capsule endoscope 2 in the subject. Specifically, in the magnetic-field generating device 3, a three-axis orthogonal coordinate system (hereinafter, "absolute coordinate system") formed of orthogonal three axes (X-axis, Y-axis, and Z-axis) is defined, to generate a magnetic field of a desired strength, respectively, with respect to respective axial directions (X-axis direction, Y-axis direction, and Z-axis direction) of the absolute coordinate system. In the magnetic-field generating device 3, a subject (not shown) supported by a bed or the like is positioned inside a three-dimensional space S (a space surrounded by a plurality of electromagnets of the magnetic-field generating device 3) of the absolute coordinate system, and a three-dimensional rotating magnetic field or three-dimensional gradient magnetic field formed by the magnetic fields in the respective axial directions of the absolute coordinate system is applied to the capsule endoscope 2 inside the subject. Accordingly, the magnetic-field generating device 3 moves the magnet in the capsule endoscope 2, and magnetically guides the capsule endoscope 2 to a desired position in the subject, while causing the capsule endoscope 2 to perform a desired operation (a forward/backward operation, a parallel shifting operation, a rotating operation, a direction changing operation or the like) following the operation of the magnet. The magnetic fields in the respective axial directions of the absolute coordinate system to be generated by the magnetic-field generating device 3 (that is, the rotating magnetic field and gradient magnetic field) are controlled by an alternating current supplied from the coil power source 4 (an energization amount from the coil power source 4).

The absolute coordinate system can be the three-axis orthogonal coordinate system defined with respect to the magnetic-field generating device 3 (that is, fixed to the magnetic-field generating device 3); however, the absolute coordinate system can be a three-axis orthogonal coordinate system fixed to the subject (not shown) incorporating the capsule endoscope 2 in the digestive canal, or a three-axis orthogonal coordinate system fixed to a support body (not shown) such as a bed that supports the subject.

The coil power source 4 supplies a current for generating the magnetic field to be applied to the capsule endoscope 2 in the subject to the magnetic-field generating device 3. The coil power source 4 supplies an alternating current to a plurality of electromagnets (coils) in the magnetic-field generating device 3 under control of the control device 11, to generate the magnetic fields in the respective axial directions of the absolute coordinate system.

A plurality of the receiving antennas 5 capture the radio signal from the capsule endoscope 2 introduced into the subject. Specifically, the receiving antennas 5 are appropriately distributed and arranged on the body surface of the subject introducing the capsule endoscope 2 into the digestive canal, to capture the radio signal from the capsule endoscope 2 moving along the digestive canal. The receiving antennas 5 transmit the radio signal from the capsule endoscope 2 to the receiving unit 6. The radio signal from the capsule endoscope 2 corresponds to an image signal including the in-vivo image of the subject captured by the capsule endoscope 2.

The receiving unit 6 is connected to the receiving antennas 5, to receive the radio signal from the capsule endoscope 2 via the receiving antennas 5. In this case, the receiving unit 6 selects a receiving antenna having the highest reception field strength from the receiving antennas 5, and performs a demodulating process or the like with respect to the radio signal received from the capsule endoscope 2 via the selected receiving antenna to demodulate the image signal corresponding to the radio signal. The receiving unit 6 transmits the demodulated image signal to the control device 11. The image signal demodulated by the receiving unit 6 includes the in-vivo image of the subject captured by the capsule endoscope 2.

The input unit 7 is realized by using an input device such as a keyboard and a mouse, and inputs various pieces of information to the control device 11 in response to an input operation performed by a user such as a doctor or a nurse. The various pieces of information input by the input unit 7 to the control device 11 include, for example, instruction information for giving instructions to the control device 11, patient information of the subject, and examination information of the subject. The instruction information with respect to the control device 11 includes instruction information for instructing magnetic guidance of the capsule endoscope 2 introduced into the subject, instruction information for displaying the various pieces of information such as the in-vivo image on the display unit 8, and instruction information for instructing reception start or reception end of the radio signal from the capsule endoscope 2 by the receiving unit 6.

The patient information of the subject is specific information for specifying the subject, and includes, for example, a patient name, a patient ID, the date of birth, sex, and age of the subject. The examination information of the subject is specific information for specifying a capsule endoscopic examination performed with respect to the subject (an examination for observing the inside of the digestive canal by introducing the capsule endoscope into the digestive canal), and includes, for example, an examination ID and an examination date.

The display unit 8 is realized by using various types of displays such as a CRT display or a liquid crystal display, to display various pieces of information instructed to be displayed by the control device 11. Specifically, the display unit 8 displays information useful for the capsule endoscopic examination such as an in-vivo image group of the subject captured by the capsule endoscope 2, the patient information of the subject, and the examination information of the subject. The display unit 8 also displays information useful for magnetic guidance of the capsule endoscope 2 such as position information of the capsule endoscope 2 in the subject.

The storage unit 9 is realized by using various types of storage media for rewritably storing information such as a RAM, an EEPROM, a flash memory, or a hard disk. The storage unit 9 stores various pieces of information instructed to be stored by the control device 11, and transmits information instructed to be read by the control device 11 from the stored various pieces of information to the control device 11. The storage unit 9 stores the in-vivo image group of the subject, the patient information and examination information of the subject, and position information of the capsule endoscope 2 detected by the position detecting device 10 under control of the control device 11.

The position detecting device 10 three-dimensionally detects a position and an orientation (a direction) of the capsule endoscope 2 inside the three-dimensional space of the absolute coordinate system. Specifically, the position detecting device 10 applies the magnetic field to the LC marker 2a of the capsule endoscope 2 positioned inside the three-dimensional space of the absolute coordinate system to cause the LC marker 2a to emit the induced magnetic field, and detects the induced magnetic field generated from the LC marker 2a. Every time the position detecting device 10 acquires a detection result of the induced magnetic field detected from the LC marker 2a, the position detecting device 10 calculates space coordinates and a directional vector of the capsule endoscope 2 (for example, a directional vector in a longitudinal axis direction of the capsule endoscope 2) in the absolute coordinate system, based on the acquired detection result of the induced magnetic field. The position detecting device 10 three-dimensionally detects the position information of the capsule endoscope 2 in the subject based on the space coordinates and directional vector of the capsule endoscope 2 in the absolute coordinate system. The position detecting device 10 transmits the position information of the capsule endoscope 2 detected in this manner to the control device 11. The position information detected by the position detecting device 10 includes various pieces of information of a current position and a current orientation (direction) of the capsule endoscope 2 in the subject.

The orientation of the capsule endoscope 2 is determined by the longitudinal axis direction of a capsule casing included in the capsule endoscope 2, and a rotation state around a longitudinal axis of the capsule endoscope 2 specified by a radial direction of the capsule casing (orthogonal two-axis directions perpendicular to the longitudinal axis direction).

The control device 11 controls the operation of respective components (the magnetic-field generating device 3, the coil power source 4, the receiving unit 6, the input unit 7, the display unit 8, the storage unit 9, and the position detecting device 10) of the capsule guidance system 1, and controls the input/output of the signal between the respective components. Specifically, the control device 11 controls respective operations of the receiving unit 6, the position detecting device 10, the display unit 8, and the storage unit 9 based on the instruction information input by the input unit 7. The control device 11 also controls the energization amount of the coil power source 4 with respect to the magnetic-field generating device 3 based on the instruction information input by the input unit 7, and controls a magnetic-field generating operation of the magnetic-field generating device 3 through control of the coil power source 4. Accordingly, the control device 11 controls magnetic guidance of the capsule endoscope 2 in the subject. The control device 11 controls an operation timing of the magnetic-field generating device 3 and an operation timing of the receiving unit 6 so that the timing for the magnetic-field generating device 3 to apply the magnetic field to the capsule endoscope 2 and the timing for the receiving unit 6 to receive the radio signal from the capsule endoscope 2 does not overlap on each other.

The control device 11 acquires position information of the capsule endoscope 2 from the position detecting device 10, to display the acquired position information (that is, the current position and orientation of the capsule endoscope 2 in the subject) on the display unit 8. The control device 11 controls the display unit 8 so that the display unit 8 updates the current position and orientation of the capsule endoscope 2 in the subject, every time the position information of the capsule endoscope 2 is acquired from the position detecting device 10.

Further, the control device 11 has an image processing function for generating (reconstructing) the in-vivo image of the subject based on the image signal demodulated by the receiving unit 6. Specifically, the control device 11 acquires the image signal from the receiving unit 6, and performs predetermined image processing with respect to the acquired image signal to generate image information (that is, the in-vivo image of the subject captured by the capsule endoscope 2). The control device 11 sequentially stores the in-vivo image of the subject generated in this manner in the storage unit 9, and displays the in-vivo image of the subject on the display unit 8 based on the instruction information from the input unit 7.

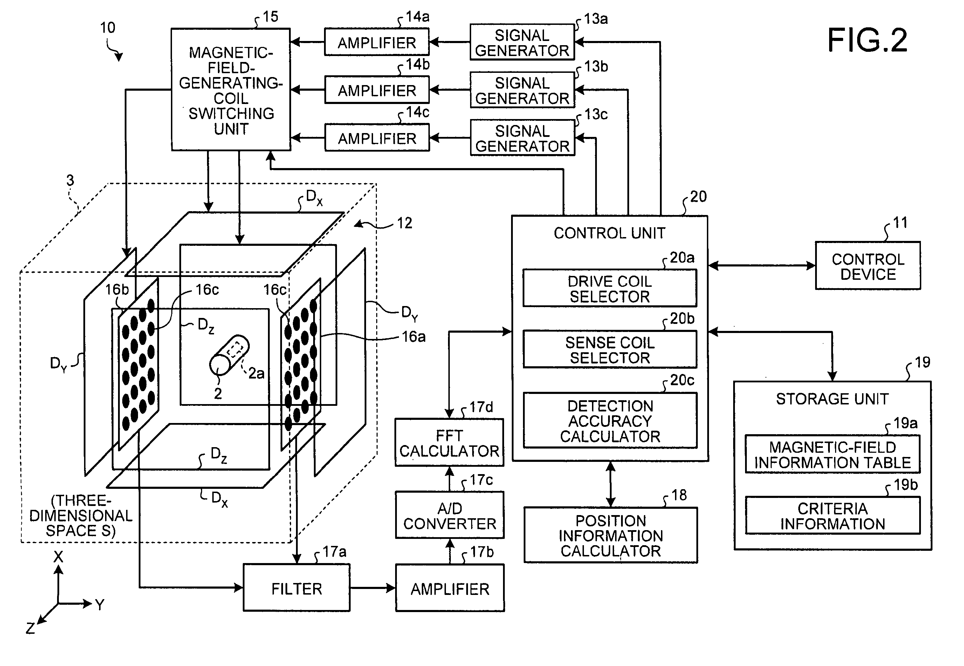

The position detecting device 10 according to the present embodiment of the present invention is explained next. FIG. 2 is a schematic block diagram of a configuration example of the position detecting device 10 according to the present embodiment of the present invention. As shown in FIG. 2, the position detecting device 10 according to the present embodiment includes a magnetic field generator 12 that generates the magnetic field to be applied to the LC marker 2a in the capsule endoscope 2, signal generators 13a to 13c that generate alternating-current signals to be applied to the magnetic field generator 12, amplifiers 14a to 14c that respectively amplify each alternating-current signal output from the signal generators 13a to 13c, and a magnetic-field-generating-coil switching unit 15 that switches magnetic-field generating coils (drive coils D_{X}, D_{Y}, and D_{Z} described later) of the magnetic field generator 12 that generates the magnetic field with respect to the LC marker 2a in the capsule endoscope 2. The position detecting device 10 further includes magnetic field detectors 16a and 16b that detect the induced magnetic field generated from the LC marker 2a in the capsule endoscope 2, a filter 17a that removes unnecessary frequency components included in an output signal from the magnetic field detectors 16a and 16b, an amplifier 17b that amplifies an output signal from the filter 17a, an A/D converter 17c that converts an analog signal amplified by the amplifier 17b to a digital signal, an FFT calculator 17d that performs Fast Fourier Transform (FFT processing) with respect to the digital signal digitized by the A/D converter 17c, and a position information calculator 18 that calculates position information of the capsule endoscope 2 based on the digital signal FFT-processed by the FFT calculator 17d. The position detecting device 10 further includes a storage unit 19 that stores various pieces of information, and a control unit 20 that controls respective components of the position detecting device 10.

The magnetic field generator 12 is arranged in an internal space of the magnetic-field generating device 3, that is, the three-dimensional space S of the absolute coordinate system, to generate the magnetic field to be applied to the LC marker 2a in the capsule endoscope 2 positioned in the three-dimensional space S. Specifically, the magnetic field generator 12 is realized by combining a plurality of the drive coils D_{X}, D_{Y}, and D_{Z} as exemplified by a Helmholtz coil.

A set of the drive coils D_{X} is realized by a pair of coils facing the X-axis direction of the absolute coordinate system, to generate an alternating magnetic field in the X-axis direction of the absolute coordinate system, and applies the generated alternating magnetic field in the X-axis direction to the LC marker 2a, thereby generating the induced magnetic field in the LC marker 2a. A set of the drive coils D_{Y} is realized by a pair of coils facing the Y-axis direction of the absolute coordinate system, to generate an alternating magnetic field in the Y-axis direction of the absolute coordinate system, and applies the generated alternating magnetic field in the Y-axis direction to the LC marker 2a, thereby generating the induced magnetic field in the LC marker 2a. A set of the drive coils D_{Z} is realized by a pair of coils facing the Z-axis direction of the absolute coordinate system, to generate an alternating magnetic field in the Z-axis direction of the absolute coordinate system, and applies the generated alternating magnetic field in the Z-axis direction to the LC marker 2a, thereby generating the induced magnetic field in the LC marker 2a. A active state of plural sets of the drive coils D_{X}, D_{Y}, and D_{Z} (that is, a state that the magnetic field is generated with respect to the LC marker 2a) is switched by the magnetic-field-generating-coil switching unit 15 described later. The magnetic field generated by any one of the sets of the drive coils D_{X}, D_{Y}, and D_{Z} has a magnetic field component penetrating a coil axis direction of the LC marker 2a in the three-dimensional space S, thereby generating the induced magnetic field in the LC marker 2a.

The signal generators 13a to 13c respectively generate the alternating-current signal to be applied to plural sets of the drive coils D_{X}, D_{Y}, and D_{Z} in the magnetic field generator 12. Specifically, the signal generator 13a generates the alternating-current signal to be applied to a set of the drive coils D_{X} under control of the control unit 20, and outputs the generated alternating-current signal to the amplifier 14a. The signal generator 13b generates the alternating-current signal to be applied to a set of the drive coils D_{Y} under control of the control unit 20, and outputs the generated alternating-current signal to the amplifier 14b. The signal generator 13c generates the alternating-current signal to be applied to a set of the drive coils D_{Z} under control of the control unit 20, and outputs the generated alternating-current signal to the amplifier 14c.

The amplifiers 14a to 14c respectively amplify each alternating-current signal output by the signal generators 13a to 13c. Specifically, the amplifier 14a amplifies the alternating-current signal output by the signal generator 13a (alternating-current signal to be applied to a set of the drive coils D_{X}), and outputs the amplified alternating-current signal to the magnetic-field-generating-coil switching unit 15. The amplifier 14b amplifies the alternating-current signal output by the signal generator 13b (alternating-current signal to be applied to a set of the drive coils D_{Y}), and outputs the amplified alternating-current signal to the magnetic-field-generating-coil switching unit 15. The amplifier 14c amplifies the alternating-current signal output by the signal generator 13c (alternating-current signal to be applied to a set of the drive coils D_{Z}), and outputs the amplified alternating-current signal to the magnetic-field-generating-coil switching unit 15.

The magnetic-field-generating-coil switching unit 15 switches the active state of the magnetic field generator 12 that applies the magnetic field to the LC marker 2a in the capsule endoscope 2 positioned in the three-dimensional space S. Specifically, the magnetic-field-generating-coil switching unit 15 has a relay that switches a connecting state and a non-connecting state between the signal generator 13a and a set of the drive coils D_{X} (hereinafter, "relay of the drive coils D_{X}"), a relay that switches a connecting state and a non-connecting state between the signal generator 13b and a set of the drive coils D_{Y} (hereinafter, "relay of the drive coils D_{Y}"), and a relay that switches a connecting state and a non-connecting state between the signal generator 13c and a set of the drive coils D_{Z} (hereinafter, "relay of the drive coils D_{Z}"), and respectively switches an ON/OFF state of the relay under control of the control unit 20.

More specifically, when the relay of the drive coils D_{X} is turned to the ON state, the magnetic-field-generating-coil switching unit 15 connects a set of the drive coils D_{X} and the signal generator 13a with each other via the amplifier 14a, and applies the alternating-current signal amplified by the amplifier 14a (that is, the alternating-current signal generated by the signal generator 13a) to a set of the drive coils D_{X}. In this case, the magnetic-field-generating-coil switching unit 15 switches a set of the drive coils D_{X} to an active state (a state that the magnetic field is applied to the LC marker 2a). When the relay of the drive coils D_{X} is turned to the OFF state, the magnetic-field-generating-coil switching unit 15 disconnects between the set of the drive coils D_{X} and the signal generator 13a, to switch the set of the drive coils D_{X} to a non-active state (a state that the magnetic field is not generated).

On the other hand, when the relay of the drive coils D_{Y} is turned to the ON state, the magnetic-field-generating-coil switching unit 15 connects a set of the drive coils D_{Y} and the signal generator 13b with each other via the amplifier 14b, and applies the alternating-current signal amplified by the amplifier 14b (that is, the alternating-current signal generated by the signal generator 13b) to a set of the drive coils D_{Y}. In this case, the magnetic-field-generating-coil switching unit 15 switches the set of the drive coils D_{Y} to an active state. When the relay of the drive coils D_{Y} is turned to the OFF state, the magnetic-field-generating-coil switching unit 15 disconnects between the set of the drive coils D_{Y} and the signal generator 13b, to switch the set of the drive coils D_{Y} to a non-active state.

On the other hand, when the relay of the drive coils D_{Z} is turned to the ON state, the magnetic-field-generating-coil switching unit 15 connects a set of the drive coils D_{Z} and the signal generator 13c with each other via the amplifier 14c, and applies the alternating-current signal amplified by the amplifier 14c (that is, the alternating-current signal generated by the signal generator 13c) to a set of the drive coils D_{Z}. In this case, the magnetic-field-generating-coil switching unit 15 switches a set of the drive coils D_{Z} to an active state. When the relay of the drive coils D_{Z} is turned to the OFF state, the magnetic-field-generating-coil switching unit 15 disconnects between the set of the drive coils D_{Z} and the signal generator 13c, to switch the set of the drive coils D_{Z} to a non-active state.

The magnetic field detectors 16a and 16b detect the induced magnetic field generated from the LC marker 2a in the capsule endoscope 2 by reacting to the magnetic field from the outside. Specifically, the magnetic field detectors 16a and 16b respectively have, for example, a plurality of sense coils 16c arranged in a matrix, and are arranged opposite to each other in an internal space surrounded by the plural sets of the drive coils D_{X}, D_{Y}, and D_{Z}. Each of the sense coils 16c of the magnetic field detectors 16a and 16b respectively detects the induced magnetic field generated from the LC marker 2a by reacting to any of the magnetic fields of the plural sets of the drive coils D_{X}, D_{Y}, and D_{Z}, and outputs a field strength signal corresponding to the field strength of the detected induced magnetic field to the filter 17a as a detection result of the induced magnetic field. In the present embodiment, the field strength signal is used as the detection result of the sense coil 16c. However, the detection result is not limited to the field strength. For example, magnetic field information such as a phase of the magnetic field can be used as the detection result of the sense coil 16c.

The filter 17a removes unnecessary frequency components included in the field strength signal output from each of the sense coils 16c of the magnetic field detectors 16a and 16b and transmits the field strength signal with the unnecessary frequency components being removed to the amplifier 17b. The amplifier 17b amplifies the field strength signal, which is an output signal from the filter 17a, and outputs the amplified field strength signal to the A/D converter 17c. The A/D converter 17c converts the field strength signal (an analog signal) amplified by the amplifier 17b to a digital signal, and transmits the digitized field strength signal to the FFT calculator 17d. The FFT calculator 17d performs predetermined FFT processing with respect to the field strength signal digitized by the A/D converter 17c, and transmits an FFT processing result (that is, a detection result of the field strength of the induced magnetic field generated from the LC marker 2a) to the control unit 20. The FFT processing result is used for a position information calculating process of the capsule endoscope 2 performed by the position information calculator 18.

The position information calculator 18 calculates the position information of the capsule endoscope 2 in the subject based on the detection result of the induced magnetic field of the LC marker 2a detected by each of the sense coils 16c of the magnetic field detectors 16a and 16b. Specifically, the position information calculator 18 calculates the position information of the LC marker 2a in the three-dimensional space S based on the detection result of the field strength acquired by each sense coil (that is, the FFT processing result by the FFT calculator 17d) selected by the control unit 20 from a plurality of the sense coils 16c. Accordingly, the position information calculator 18 calculates the position information in the subject of the capsule endoscope 2 incorporating the LC marker 2a. The position information of the capsule endoscope 2 calculated by the position information calculator 18 includes various pieces of information of the current position and orientation (direction) of the capsule endoscope 2 (in more detail, the LC marker 2a) in the subject. The position information calculator 18 transmits the calculated position information of the capsule endoscope 2 to the control unit 20.

The storage unit 19 stores the various pieces of information under control of the control unit 20. Specifically, the storage unit 19 stores a magnetic-field information table 19a and criteria information 19b as predetermined information of the selecting condition for selecting the drive coil (any of the drive coils D_{X}, D_{Y}, and D_{Z}) in an active state by the control unit 20, which is to be switched by the magnetic-field-generating-coil switching unit 15. The magnetic-field information table 19a is a look-up table indicating respective pieces of field strength information generated, respectively, by plural sets of the drive coils D_{X}, D_{Y}, and D_{Z} at respective positions on the X-axis, the Y-axis, and the Z-axis of the absolute coordinate system. The criteria information 19b includes a coefficient A less than 1 (hereinafter, "hysteresis coefficient A") used for the control unit 20 to hysteretically select drive coils in an active state to be switched by the magnetic-field-generating-coil switching unit 15 from the plural sets of the drive coils D_{X}, D_{Y}, and D_{Z}. The storage unit 19 stores the information instructed to be stored by the control unit 20, for example, the position information of the capsule endoscope 2 calculated by the position information calculator 18 and transmits the information instructed to be read by the control unit 20 to the control unit 20.

The field strength information included in the magnetic-field information table 19a desirably includes an influence of peripheral interference. The influence of peripheral interference in this context includes a magnetic field caused by electromagnetic induction between respective coils in the magnetic-field generating device 3 that generates the magnetic field for magnetically guiding the capsule endoscope 2 or coils such as wireless feeding coils (not shown) that supply power to the capsule endoscope 2, and a change of magnetic field distribution due to a magnetic body (for example, a magnet contained in the capsule endoscope 2) or metal.

The control unit 20 controls the operation of the respective components of the position detecting device 10 (the signal generators 13a to 13c, the amplifiers 14a to 14c, the magnetic-field-generating-coil switching unit 15, the magnetic field detectors 16a and 16b, the filter 17a, the amplifier 17b, the A/D converter 17c, the FFT calculator 17d, the position information calculator 18, and the storage unit 19), and controls the input/output of signals between the respective components. The control unit 20 also controls the drive of the respective drive coils of the magnetic field generator 12 (generation of the magnetic field with respect to the LC marker 2a) through control of generation of the alternating-current signal by the signal generators 13a to 13c. The control unit 20 controls the respective components of the position detecting device 10 based on the instruction from the control device 11, to acquire the position information of the LC marker 2a in the three-dimensional space S, that is, the position information of the capsule endoscope 2 in the subject. In this case, the control unit 20 synchronizes the operation of the magnetic-field-generating-coil switching unit 15 with the operation of the FFT calculator 17d, and stops the FFT processing of the FFT calculator 17d and the detection operation of the sense coil 16c, during a period where the magnetic-field-generating-coil switching unit 15 switches the drive coils in an active state. Alternatively, the operation of the A/D converter 17c and the detection operation of the sense coil 16c can be stopped. It can be regarded that the detection operation of the sense coil 16c is stopped by determining the FFT calculation result during the period of switching the drive coils in an active state invalid by the control device 11 or the position information calculator 18. The position information of the capsule endoscope 2 acquired by the control unit 20 (that is, detected by the position detecting device 10) is transmitted from the control unit 20 to the control device 11, and displayed on the display unit 8 as described above.

The control unit 20 includes a drive coil selector 20a that selects drive coils in an active state from the plural sets of the drive coils D_{X}, D_{Y}, and D_{Z}, a sense coil selector 20b that selects a sense coil to be used for a position information calculating process of the capsule endoscope 2 from the sense coils 16c, and a detection accuracy calculator 20c that calculates detection accuracy of the position information of the capsule endoscope 2.

The drive coil selector 20a determines the selecting condition of the drive coil based on the magnetic-field information table 19a and the criteria information 19b in the storage unit 19, to select a set of drive coils that satisfies the selecting condition from the plural sets of the drive coils D_{X}, D_{Y}, and D_{Z}. Accordingly, the drive coil selector 20a hysteretically selects a set of drive coils that applies the magnetic field to the LC marker 2a in the capsule endoscope 2 positioned in the three-dimensional space S. The control unit 20 controls the signal generators 13a to 13c and the magnetic-field-generating-coil switching unit 15 so that the magnetic field is generated by the set of drive coils hysteretically selected by the drive coil selector 20a.

The sense coil selector 20b selects a sense coil to be used for the position information calculating process of the capsule endoscope 2 from the sense coils 16c included in the magnetic field detectors 16a and 16b. Specifically, the sense coil selector 20b invalidates a sense coil in which a strength detection value of the magnetic field generated by the set of drive coils selected by the drive coil selector 20a is saturated and a sense coil in which the strength detection value of the magnetic field generated by the set of drive coils is substantially zero, to select a sense coil valid for the position information calculating process of the capsule endoscope 2 from the sense coils 16c.

The detection result of the field strength of the respective sense coils 16c includes a strength detection value of the induced magnetic field of the LC marker 2a and a strength detection value of the magnetic field generated from a set of drive coils (any of the sets of the drive coils D_{X}, D_{Y}, and D_{Z}) in an active state. That is, the strength detection value of the magnetic field from the drive coils detected by each of the sense coil 16c is a reference value (hereinafter, "calibration value") for extracting the strength detection value of the induced magnetic field of the LC marker 2a from the detection result of the field strength of each of the sense coil 16c. The sense coil selector 20b invalidates a sense coil having a calibration value inappropriate for the position information calculating process of the capsule endoscope 2, that is, a sense coil in which the calibration value is saturated or substantially zero, of the sense coils 16c, to select the remaining valid sense coils. The control unit 20 uses the detection result of the field strength of the respective valid sense coils selected by the sense coil selector 20b and controls the position information calculator 18 to calculate the position information of the capsule endoscope 2.

The position information calculator 18 subtracts the calibration value from the detection result of the field strength of the respective valid sense coils 16c to calculate the strength detection value of the induced magnetic field of the LC marker 2a acquired by the respective sense coils 16c, and calculates the position information of the capsule endoscope 2 based on the acquired strength detection value of the respective sense coils 16c.

The detection accuracy calculator 20c calculates the detection accuracy of the position information of the capsule endoscope 2 (that is, the current position information and current direction information of the capsule endoscope 2) based on a theoretical value of the field strength of the magnetic field generated by the drive coils in an active state at the current position of the capsule endoscope 2 and a noise amount presumed by the position detecting device 10. In this case, the detection accuracy calculator 20c calculates a strength theoretical value of the magnetic field at the current position of the capsule endoscope 2 based on the magnetic-field information table 19a in the storage unit 19 and the position information calculation result of the position information calculator 18. The detection accuracy calculator 20c calculates the detection accuracy by calculating an error in the respective pieces of information of the current position and direction of the capsule endoscope 2 based on the strength theoretical value and the noise amount of the entire position detecting device 10. A detection accuracy calculation result calculated by the detection accuracy calculator 20c is transmitted from the control unit 20 to the control device 11. As a result, it can be known how much trust can be placed (that is, how much error is included) in the position information of the capsule endoscope 2 detected by the position detecting device 10 according to the present invention.

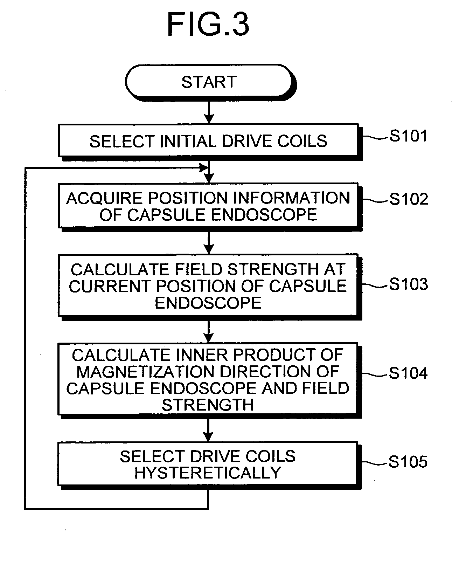

A process procedure performed by the drive coil selector 20a that hysteretically selects drive coils in an active state from the plural sets of the drive coils D_{X}, D_{Y}, and D_{Z} (that is, a set of drive coils that applies the magnetic field to the LC marker 2a in the capsule endoscope 2 positioned in the three-dimensional space S) is explained below. FIG. 3 is a flowchart for exemplifying the process procedure performed by the drive coil selector 20a that hysteretically selects drive coils in an active state.

As shown in FIG. 3, the drive coil selector 20a first selects initial drive coils to be driven first from plural sets of the drive coils D_{X}, D_{Y}, and D_{Z} of the magnetic field generator 12 (Step S101). Specifically, the drive coil selector 20a selects first drive coils for applying the magnetic field to the LC marker 2a in the capsule endoscope 2 (that is, the capsule endoscope 2 introduced into internal organs of the subject) positioned in the three-dimensional space S of the absolute coordinate system from the plural sets of the drive coils D_{X}, D_{Y}, and D_{Z}. In this case, the drive coil selector 20a initially selects drive coils specified in advance by taking into consideration a coil axis direction of the LC marker 2a at the time of introducing the capsule endoscope 2 into the internal organs of the subject (for example, drive coils capable of forming a magnetic field penetrating the LC marker 2a in the coil axis direction). In this case, the control unit 20 controls the signal generators 13a to 13c and the magnetic-field-generating-coil switching unit 15 so that the initial drive coils applies the magnetic field to the LC marker 2a.

The drive coil selector 20a then acquires the position information of the capsule endoscope 2 calculated by the position information calculator 18 (Step S102). In this case, the drive coil selector 20a acquires a current position coordinate P_{C} = (x, y, z) and a current orientation M_{C} = (mx, my, mz) of the capsule endoscope 2 in the absolute coordinate system as the position information of the capsule endoscope 2. The current orientation M_{C} = (mx, my, mz) is a current direction of the capsule endoscope 2 in the subject and is a magnetization direction (a coil axis direction) of the LC marker 2a.

Subsequently, the drive coil selector 20a calculates the field strength at the current position of the capsule endoscope 2 (Step S103). At Step S103, the drive coil selector 20a calculates an X-axis component, a Y-axis component, and a Z-axis component of respective field strengths generated by each set of drive coils at the current position of the capsule endoscope 2, based on the position information of the capsule endoscope 2 and the magnetic-field information table 19a. Specifically, the drive coil selector 20a performs predetermined interpolation processing by using the respective field strengths indicated in the magnetic-field information table 19a (field strength at respective positions on the X-axis, field strength at respective positions on the Y-axis, and field strength at respective positions on the Z-axis) and the current position coordinate P_{C} = (x, y, z) of the capsule endoscope 2, to thereby calculate field strength B_{DX} = (B_{xDX}, B_{yDX}, B_{zDX}) to be generated in the current position coordinate P_{C} by a set of the drive coils D_{X}, field strength B_{DY} = (B_{xDY}, B_{yDY}, B_{zDY}) to be generated in the current position coordinate P_{C} by a set of drive coils D_{Y}, and field strength B_{DZ} = (B_{xDZ}, B_{yDZ}, B_{zDZ}) to be generated in the current position coordinate P_{C} by a set of the drive coils D_{Z}.

Thereafter, the drive coil selector 20a calculates an inner product of the magnetization direction of the capsule endoscope 2 and the field strength from the drive coils (Step S104). At Step S104, the drive coil selector 20a calculates field strengths E_{DX}, E_{DY}, and E_{DZ}, with which the induced magnetic field is to be generated in the LC marker 2a positioned at the current position coordinate P_{C}, according to an inner product of the magnetization direction of the capsule endoscope 2, that is, the current orientation M_{C} = (mx, my, mz) and the field strengths B_{DX}, B_{DY}, and B_{DZ} of the respective drive coils.

The field strength B_{DX} (=|M_{C}·B_{DX}|) is a strength of a magnetic field component that generates the induced magnetic field in the LC marker 2a (that is, a magnetic field component penetrating the LC marker 2a in the coil axis direction), of the magnetic field components to be applied to the LC marker 2a positioned at the current position coordinate P_{C} by a set of the drive coils D_{X}. Likewise, the field strength B_{DY} (=|M_{C}·B_{DY}|) is a strength of a magnetic field component that generates the induced magnetic field in the LC marker 2a, of the magnetic field components to be applied to the LC marker 2a positioned at the current position coordinate P_{C} by a set of drive coils D_{Y}, and the field strength B_{DZ} (=|M_{C}·B_{DZ}|) is a strength of a magnetic field component that generates the induced magnetic field in the LC marker 2a, of the magnetic field components to be applied to the LC marker 2a positioned at the current position coordinate P_{C} by a set of the drive coils D_{Z}.

The drive coil selector 20a then determines the selecting condition of the drive coil based on the magnetic-field information table 19a and the criteria information 19b, to hysteretically select drive coils that satisfy the selecting condition from plural sets of the drive coils D_{X}, D_{Y}, and D_{Z} (Step S105). In this case, the drive coil selector 20a multiplies the field strengths E_{DX}, E_{DY}, and E_{DZ} calculated at Step S104 by the hysteresis coefficient A, respectively, to calculate thresholds associated with the field strength, and selects a set of drive coils corresponding to a field strength E_{P} (any of the field strengths E_{DX}, E_{DY}, and E_{DZ}) larger than the calculated respective thresholds.

Specifically, the drive coil selector 20a multiplies respective field strengths Eₙₚ₁ and Eₙₚ₂ (any two of the field strengths E_{DX}, E_{DY}, and E_{DZ}) of the drive coils in a non-active state (that is, in a state that the magnetic field is not generated) of the field strengths E_{DX}, E_{DY}, and E_{DZ} by the hysteresis coefficient A, respectively, to calculate a threshold with respect to the field strength E_{P} of the drive coils in an active state, which is actually applying the magnetic field to the LC marker 2a. The drive coil selector 20a compares the two thresholds (Eₙₚ₁ × A, Eₙₚ₂ × A) with the field strength E_{P}, and when the field strength E_{P} is larger than the respective thresholds, that is, E_{P} > Eₙₚ₁ × A and E_{P} > Eₙₚ₂ × A, the drive coil selector 20a selects drive coils D_{P} currently in an active state corresponding to the field strength E_{P}. In this case, the control unit 20 controls the signal generators 13a to 13c and the magnetic-field-generating-coil switching unit 15 to maintain the active state of the drive coils D_{P} and the non-active state of other drive coils Dₙₚ₁ and Dₙₚ₂.

When the drive coils D_{P} in an active state are the drive coils D_{X}, the drive coils Dₙₚ₁ and Dₙₚ₂ in a non-active state are remaining drive coils D_{Y} and D_{Z}. When the drive coils D_{P} in an active state are the drive coils D_{Y}, the drive coils Dₙₚ₁ and Dₙₚ₂ in a non-active state are the remaining drive coils D_{X} and D_{Z} and when the drive coils D_{P} in an active state are the drive coils D_{Z}, the drive coils Dₙₚ₁ and Dₙₚ₂ in a non-active state are the remaining drive coils D_{X} and D_{Y}.

On the other hand, when the drive coil selector 20a compares the two thresholds (Eₙₚ₁ × A, Eₙₚ₂ × A) with the field strength E_{P}, and the field strength E_{P} is equal to or smaller than the threshold (Eₙₚ₁ × A) and larger than the threshold Eₙₚ₂ × A (E_{P} ≤ Eₙₚ₁ × A and E_{P} > Eₙₚ₂ × A), the drive coil selector 20a selects drive coils Dₙₚ₁ in a non-active state corresponding to the field strength Eₙₚ₁, instead of the drive coils D_{P} corresponding to the field strength E_{P}. In this case, the control unit 20 controls the signal generators 13a to 13c and the magnetic-field-generating-coil switching unit 15 to release an active state of the drive coils D_{P} to switch the drive coils Dₙₚ₁ to an active state, and switch the drive coils D_{P} and D_{nP2} to a non-active state.

On the other hand, when the drive coil selector 20a compares the two thresholds (Eₙₚ₁ × A, Eₙₚ₂ × A) with the field strength E_{P}, and the field strength E_{P} is larger than the threshold (Eₙₚ₁ × A) and equal to or smaller than the threshold Eₙₚ₂ × A (E_{P} > Eₙₚ₁ × A and E_{P} ≤ Eₙₚ₂ × A), the drive coil selector 20a selects the drive coil Dₙₚ₂ in a non-active state corresponding to the field strength Eₙₚ₂. In this case, the control unit 20 controls the signal generators 13a to 13c and the magnetic-field-generating-coil switching unit 15 to release the active state of the drive coils D_{P} to switch the drive coils Dₙₚ₂ to an active state, and switch the drive coils D_{P} and Dₙₚ₁ to a non-active state.

Thereafter, the drive coil selector 20a returns to step S102 to repeat the process procedure at step S102 and thereafter. Every time the drive coil selector 20a repeats the process procedure at steps S102 to S105, the control unit 20 controls the signal generators 13a to 13c and the magnetic-field-generating-coil switching unit 15 to switch a set of drive coils selected from plural sets of the drive coils D_{X}, D_{Y}, and D_{Z} selected by the drive coil selector 20a.

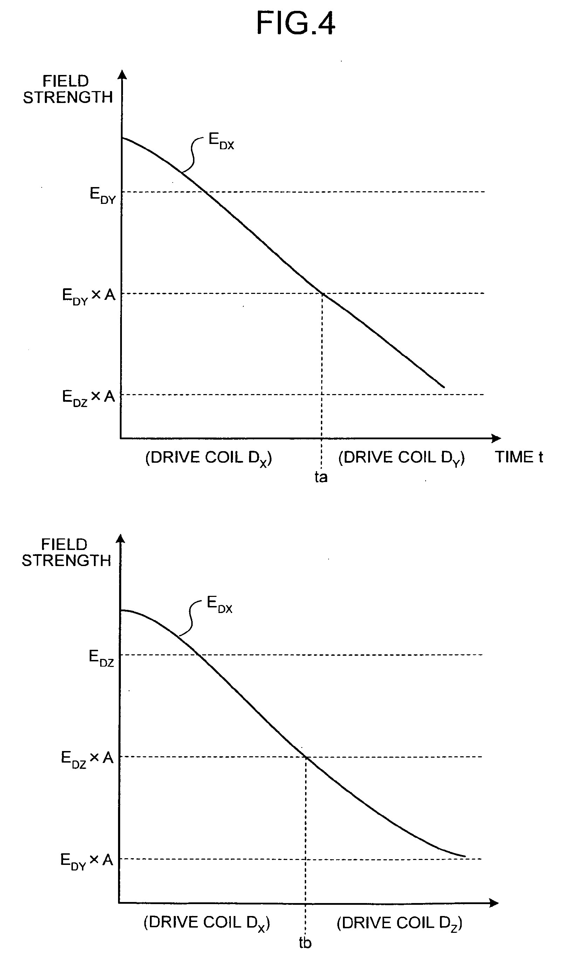

A selecting process of the drive coil by the control unit 20 is specifically explained next, by exemplifying a case that drive coils in an active state that apply the magnetic field to the LC marker 2a in the capsule endoscope 2 is switched from the drive coils D_{X} to the drive coils D_{Y} and D_{Z}. FIGS. 4 are schematic diagrams for specifically explaining an operation of the drive coil selector 20a that hysteretically selects drive coils in an active state from plural sets of the drive coils D_{X}, D_{Y}, and D_{Z}. A temporal change of the field strength E_{DX} at the time of switching drive coils that apply the magnetic field to the LC marker 2a from a set of the drive coils D_{X} to a set of the drive coils D_{Y} or a set of the drive coils D_{Z} is shown in FIGS. 4.

The drive coil selector 20a determines the selecting condition of the drive coil by using the field strengths E_{DX}, E_{DY}, and E_{DZ} to be applied to the LC marker 2a respectively by the plural sets of the drive coils D_{X}, D_{Y}, and D_{Z} and the hysteresis coefficient A, to select a set of drive coils that satisfies the determined selecting condition from the plural sets of the drive coils D_{X}, D_{Y}, and D_{Z}. For example, when the drive coils D_{X} satisfy the selecting condition of E_{P} > Eₙₚ₁ × A and E_{P} > Eₙₚ₂ × A, the drive coil selector 20a selects a set of the drive coils D_{X} that satisfies the selecting condition (that is, the drive coils having the largest field strength applied to the LC marker 2a among the plural sets of the drive coils D_{X}, D_{Y}, and D_{Z}) from the plural sets of the drive coils D_{X}, D_{Y}, and D_{Z}. The control unit 20 controls the signal generators 13a to 13c and the magnetic-field-generating-coil switching unit 15 to switch the set of the drive coils D_{X} selected by the drive coil selector 20a to an active state.

The drive coil selector 20a then calculates the field strength E_{DX} of the drive coils D_{X} in an active state and two thresholds (E_{DY} × A and E_{DZ} × A), every time the position information of the capsule endoscope 2 is acquired from the position information calculator 18, and compares the calculated field strength E_{DX} with the two thresholds (E_{DY} × A and E_{DZ} × A), to hysteretically select a set of drive coils that satisfies the selecting condition of the drive coil from the plural sets of the drive coils D_{X}, D_{Y}, and D_{Z}.

Specifically, as shown in FIGS. 4, when the field strength E_{DX} of the drive coil D_{X} currently in an active state is larger than the two thresholds (E_{DY} × A and E_{DZ} × A), the drive coil selector 20a selects a set of drive coils D_{X} that satisfies a selecting condition (E_{DX} > E_{DY} × A and E_{DX} > E_{DZ} × A). In this case, the control unit 20 controls the signal generators 13a to 13c and the magnetic-field-generating-coil switching unit 15 to maintain the active state of the set of the drive coils D_{X} and switch the other sets of the drive coils D_{Y} and D_{Z} to a non-active state.

Thereafter, even if the field strength E_{DX} of the drive coils D_{X} in an active state drops to a level equal to or lower than, for example, the field strength E_{DY} of the drive coil D_{Y} in a non-active state with a movement of the capsule endoscope 2 in the subject, the drive coil selector 20a selects the drive coils D_{X} in an active state so long as the drive coils D_{X} satisfy the selecting condition (E_{DX} > E_{DY} × A and E_{DX} > E_{DZ} × A) of the drive coil. Likewise, even if the field strength E_{DX} of the drive coils D_{X} drops to a level equal to or lower than the field strength E_{DZ} of the drive coils D_{Z} in a non-active state, the drive coil selector 20a selects the drive coils D_{X} in an active state so long as the drive coils D_{X} satisfy the selecting condition (E_{DX} > E_{DY} × A and E_{DX} > E_{DZ} × A) of the drive coil.

On the other hand, the drive coil selector 20a selects the drive coils D_{Y} in a non-active state that satisfy the selecting condition (E_{DX} > E_{DY} × A and E_{DX} > E_{DZ} × A) of the drive coil instead of the drive coils D_{X} in an active state, at a point in time when the field strength E_{DX} of the drive coils D_{X} in an active state drops to a level equal to or lower than the threshold E_{DY}×A corresponding to the drive coils D_{Y} in a non-active state. In this case, the control unit 20 controls the signal generators 13a to 13c and the magnetic-field-generating-coil switching unit 15 to release the active state of the drive coils D_{X} to switch the drive coils D_{Y} to an active state, and switch other drive coils D_{X} and D_{Z} to a non-active state.

Meanwhile, the drive coil selector 20a selects the drive coils D_{Z} in a non-active state that satisfy the selecting condition (E_{DX} > E_{DZ} × A and E_{DX} > E_{DY} × A) of the drive coil instead of the drive coils D_{X} in an active state, at a point in time when the field strength E_{DX} of the drive coils D_{X} in an active state drops to a level equal to or lower than the threshold E_{DZ} × A corresponding to the drive coils D_{Z} in a non-active state. In this case, the control unit 20 controls the signal generators 13a to 13c and the magnetic-field-generating-coil switching unit 15 to release the active state of the drive coils D_{X} to switch the drive coils D_{Z} to an active state, and switch other drive coils D_{X} and D_{Y} to a non-active state.

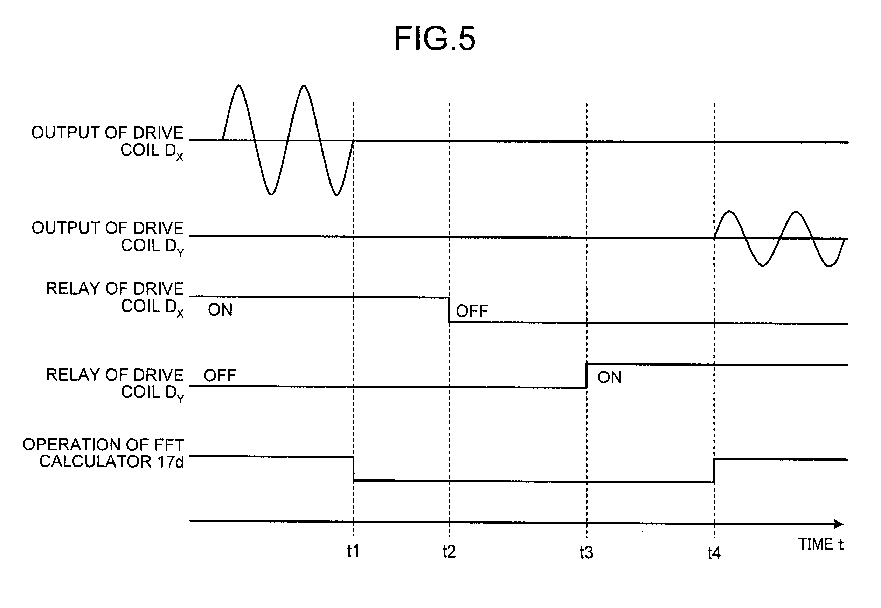

A switching control of the drive coil performed by the control unit 20 is specifically explained next, by exemplifying a case that the drive coils in an active state that apply the magnetic field to the LC marker 2a in the capsule endoscope 2 is switched from the drive coils D_{X} to the drive coils D_{Y}. FIG. 5 is a timing chart for exemplifying a switching control timing of the drive coil by the control unit 20.

As shown in FIG. 5, the control unit 20 controls the signal generator 13a at a timing (a time t1) at which the FFT calculator 17d completes the execution of the FFT processing for one section to stop an output of the alternating-current signal to the drive coils D_{X} in an active state. In this case, the control unit 20 causes the signal generator 13a to stop output of the alternating-current signal after amplitude of the alternating-current signal (sine wave) with respect to the drive coils D_{X} generated by the signal generator 13a becomes substantially zero.

Next, at a timing (a time t2) after a certain period of time has passed since suspension of output of the alternating-current signal by the signal generator 13a, the control unit 20 controls the magnetic-field-generating-coil switching unit 15 to switch the relay of the drive coils D_{X} from a connecting state (ON) to a non-connecting state (OFF). The control unit 20 can reliably exclude the alternating-current signal (a residual current) to the drive coils D_{X} remaining in the magnetic-field-generating-coil switching unit 15 or the like after the time t2 by the switching control of the relay of the drive coils D_{X}. Accordingly, it can be prevented that a back electromotive force is applied to the signal generator 13a or the amplifier 14a.

Thereafter, at a timing (a time t3) after a certain period of time has passed since switching of the relay of the drive coils D_{X} to the OFF state, the control unit 20 controls the magnetic-field-generating-coil switching unit 15 to switch the relay of the drive coils D_{Y} from the non-connecting state (OFF) to the connecting state (ON). The control unit 20 can exclude an influence of chattering of the drive coils D_{X} by the switching control from the relay of the drive coils D_{X} to the relay of the drive coils D_{Y} with an interval of the certain period of time. As a result, after the non-connecting state of the relay of the drive coils D_{X} is stabilized, the relay of the drive coils D_{Y} can be switched to the connecting state.

The control unit 20 then controls the signal generator 13b at a timing (a time t4) after a certain period of time has passed since switching the relay of the drive coils D_{Y} to the ON state, to start an output of the alternating-current signal to the drive coils D_{Y}, which are switched to an active state instead of the drive coils D_{X}. In this case, the control unit 20 causes the signal generator 13b to start an output of the alternating-current signal when the amplitude of the alternating-current signal (sine wave) to the drive coils D_{Y} generated by the signal generator 13b becomes substantially zero. The control unit 20 can exclude the influence of chattering of the drive coils D_{Y} by causing the signal generator 13b to output the alternating-current signal to the drive coils D_{Y} at the timing (the time t4) with an interval of the certain period of time, thereby enabling to exclude the influence of the chattering of the drive coils D_{Y}. As a result, after the connecting state of the relay of the drive coils D_{Y} is stabilized, the alternating-current signal from the signal generator 13b can be output to the drive coils D_{Y} via the relay of the drive coils D_{Y} (that is, the magnetic-field-generating-coil switching unit 15).

The certain period of time mentioned above, that is, a period from the time t1 to the time t2, a period from the time t2 to the time t3, and a period from the time t3 to the time t4, can be a sufficient time for excluding chattering of the respective relays in the magnetic-field-generating-coil switching unit 15 or the residual current, and the time can be acquired by experiments or the like. Abrupt generation of chattering or the like of the respective relays can be prevented by connecting the respective relays in the magnetic-field-generating-coil switching unit 15 and the respective drive coils D_{X}, D_{Y}, and D_{Z} via a low-pass filter. The respective drive coils D_{X}, D_{Y}, and D_{Z} can be stably driven (stably generate the magnetic field) by the prevention of chattering. On the other hand, at the time of starting the operation of the magnetic field generator 12, dumping or transient response can occur. The control unit 20 controls the respective signals of the signal generators 13a to 13c so that signal strength becomes constant, taking dumping or transient response into consideration, thereby enabling to perform a position detecting operation stably.

Thereafter, the control unit 20 causes the FFT calculator 17d to restart the FFT processing at the timing of the time t4. That is, the control unit 20 causes the FFT calculator 17d to stop the FFT processing at the timing (a period between the time t1 and the time t4) when the magnetic-field-generating-coil switching unit 15 is switching the drive coils in an active state. Accordingly, the control unit 20 can prevent a case that the FFT processing of the FFT calculator 17d is affected due to chattering of the relay, the residual current or the like (for example, an inappropriate value is calculated by the FFT processing) at the time of switching the drive coils in an active state by the magnetic-field-generating-coil switching unit 15. As a result, position detection accuracy of the capsule endoscope 2 at the time of switching the drive coils in an active state can be improved.

The control unit 20 applies a backup load same as that of the drive coils in an active state with respect to the signal generator 13c and the amplifier 14c corresponding to the drive coils D_{Z}, which are not directly involved with switching from the drive coils D_{X} to the drive coils D_{Y}. That is, the control unit 20 applies the backup load to the signal generator 13c and the amplifier 14c not used at the time of switching the drive coils, as exemplified in the signal generator 13c and the amplifier 14c. Accordingly, the control unit 20 can suppress the period during which the signal generators 13a to 13c and the amplifiers 14a to 14c become electrically unstable as much as possible, and the signal generator (any one of the signal generators 13a to 13c) and the amplifier (any one of the amplifiers 14a to 14c) connected to the drive coils via the magnetic-field-generating-coil switching unit 15 can perform stable operation at all times.

The control unit 20 can control the magnetic-field-generating-coil switching unit 15 to perform the switching operation of the drive coils according to an operation time. Specifically, the storage unit 19 stores a continuous operation time of the drive coil in an active state as predetermined information relating to the selecting condition for selecting drive coils in an active state (for example, any one of the drive coils D_{X}, D_{Y}, and D_{Z}). The control unit 20 can control the magnetic-field-generating-coil switching unit 15 based on the continuous operation time as the predetermined information, to continuously operate the drive coils selected from the drive coils D_{X}, D_{Y}, and D_{Z} for a certain period of time, and thereafter, to stop the drive coils in an active state for the certain period of time, and continuously operate other drive coils for the certain period of time. Alternatively, the control unit 20 can control the magnetic-field-generating-coil switching unit 15 based on the continuous operation time as the predetermined information, to operate the drive coils selected from the drive coils D_{X}, D_{Y}, and D_{Z} intermittently for a certain period of time. The time during which a current flows to the drive coil scan be reduced to a necessity minimum by performing the switching operation of the drive coils according to the operation time. As a result, a temperature rise of the drive coils in an active state can be suppressed to a certain range, to stably operate the drive coils. The position detection operation can be performed at a timing at which the temperature of the drive coils in an active state is substantially the same. As a result, the position detection operation can be performed stably at all times.

The control unit 20 can control the magnetic-field-generating-coil switching unit 15 to perform the switching operation of the drive coils according to a fixed sequence set in advance. Specifically, the storage unit 19 stores a selection sequence of the drive coils as predetermined information relating to the selecting condition for selecting the drive coils in an active state. The control unit 20 sequentially selects drive coils to be driven from the drive coils D_{X}, D_{Y}, and D_{Z} according to the selection sequence of the drive coils. The control unit 20 then controls the magnetic-field-generating-coil switching unit 15 so that the drive coils selected by the drive coil selector 20a are turned to an active state. In this case, a switching sequence of the drive coils can be a desired sequence, such as an order from the drive coils D_{X}, the drive coils D_{Y}, and the drive coils D_{Z}. The control unit 20 can cause the magnetic-field-generating-coil switching unit 15 to perform the switching operation of the drive coils according to the selection sequence for every certain period of time, or can insert the switching operation of the fixed sequence at the time of switching the drive coils based on the field strengths E_{DX}, E_{DY}, and E_{DZ}. Thus, the position detection operation can be performed at a timing at which stability of the respective drive coils is the same, by performing the switching operation of the drive coils according to the preset sequence. As a result, the position detection operation can be performed stably at all times.

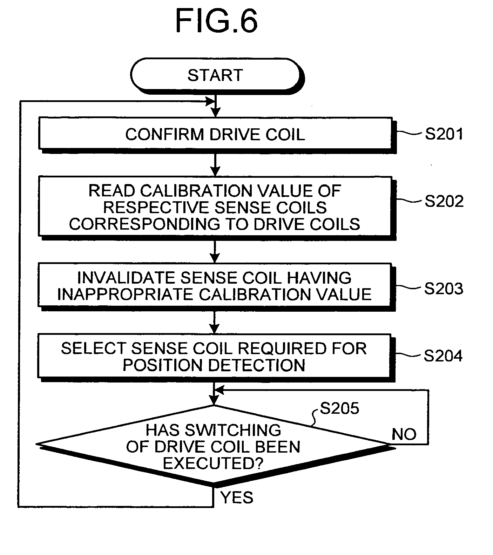

An operation of the sense coil selector 20b that selects a sense coil effective for the position information calculating process of the capsule endoscope 2 from the sense coils 16c included in the magnetic field detectors 16a and 16b is explained next. FIG. 6 is a flowchart for exemplifying a process procedure performed by the sense coil selector 20b that selects the sense coil effective for the position information calculating process of the capsule endoscope 2.

As shown in FIG. 6, the sense coil selector 20b confirms which one of the drive coils D_{X}, D_{Y}, and D_{Z} is the drive coil in an active state currently applying the magnetic field to the LC marker 2a (Step S201), and acquires a calibration value of the respective sense coils 16c corresponding to the drive coil in an active state in a state without the LC marker 2a (Step S202).

Calibration values of the respective sense coils 16c corresponding to the respective sets of the drive coils D_{X}, D_{Y}, and D_{Z} are acquired by a calibration process performed with respect to the position detecting device 10 in advance, and stored in the storage unit 19. The sense coil selector 20b reads out in advance the calibration value of the drive coil currently in an active state from the calibration values of the respective sense coils 16c stored in the storage unit 19 at Step S202.

The sense coil selector 20b confirms the calibration values of the respective sense coils 16c acquired at Step S202 and invalidates the sense coil having an inappropriate calibration value (Step S203), to select a sense coil required for position detection of the capsule endoscope 2 (Step S204). At Steps S203 and S204, the sense coil selector 20b invalidates a sense coil in which a strength detection value of the magnetic field generated from the drive coil in an active state is saturated and a sense coil in which the strength detection value of the magnetic field generated from the drive coil in an active state is substantially zero among the sense coils 16c, as the sense coil having the inappropriate calibration value, and selects the remaining sense coils other than the invalidated sense coils as the sense coil effective for the position detection of the capsule endoscope 2.

The control unit 20 transmits a detection result of the field strength of the respective effective sense coils 16c selected by the sense coil selector 20b to the position information calculator 18, and controls the position information calculator 18 to calculate the position information of the capsule endoscope 2 by using the detection result of the field strength of the respective effective sense coils 16c.

The sense coil selector 20b then determines whether switching of the drive coil in an active state has been executed (Step S205). When the drive coil in an active state is switched (YES at Step S205), control returns to Step S201, to repeat the process procedure after Step S201. On the other hand, when switching of the drive coil in an active state has not been executed (NO at Step S205), the sense coil selector 20b repeats the process at Step S205. The sense coil selector 20b selects a sense coil that can normally detect the field strength of the drive coil currently in an active state, that is, a sense coil effective for the position information calculating process of the capsule endoscope 2 from the sense coils 16c, every time the drive coil in an active state is switched.

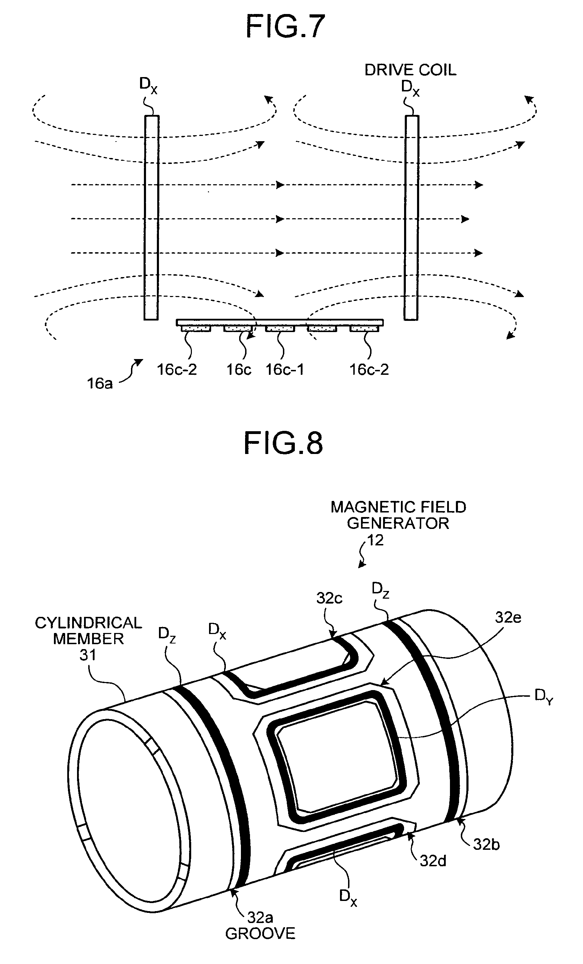

By exemplifying a case that a set of the drive coils D_{X} is in an active state, an inappropriate sense coil among the sense coils 16c in which the calibration value is saturated or substantially zero is explained. FIG. 7 is a schematic diagram for explaining of an inappropriate sense coil in which the calibration value is saturated or substantially zero.

As shown in FIG. 7, a set of the drive coils D_{X} is arranged facing each other as in the Helmholtz coil, to generate the magnetic field (an arrow with a dotted line in FIG. 7) in the X-axis direction of the absolute coordinate system. The magnetic field detector 16a is arranged at an end of a space put between the set of the drive coils D_{X}.
In this case, sense coils 16c-2 positioned near the drive coils D_{X}, of the sense coils 16c in the magnetic field detector 16a, saturate the strength detection value (that is, a calibration value) of the magnetic field, due to the strong magnetic field from the drive coils D_{X}. Because the sense coils 16c-2 in which the calibration value is saturated cannot specify the calibration value for calculating a detection result of the field strength of the induced magnetic field of the LC marker 2a, the sense coils 16c-2 are inappropriate for the position information calculating process of the capsule endoscope 2.

In a sense coil 16c-1 positioned at the same distance from the drive coils D_{X} on the opposite sides, of the sense coils 16c in the magnetic field detector 16a, the field strength is offset by the respective magnetic fields from the drive coils D_{X} on the opposite sides. Accordingly, the strength detection value (that is, the calibration value) of the magnetic field from the drive coils D_{X} acquired by the sense coil 16c-1 becomes substantially zero. Because the sense coil 16c-1 having the calibration value of substantially zero cannot accurately specify the calibration value for calculating the detection result of the field strength of the induced magnetic field of the LC marker 2a, the sense coil 16c-1 is not appropriate for the position information calculating process of the capsule endoscope 2. This is noticeable at the time of performing position detection of the capsule endoscope 2 by using the phase.

The sense coil selector 20b can prevent an adverse effect to the position information calculating process based on the detection result of the field strength of the inappropriate sense coil and increase the detection accuracy of the position information of the capsule endoscope 2, by invalidating the detection result of the field strength of the inappropriate sense coil in which the calibration value is saturated or substantially zero.

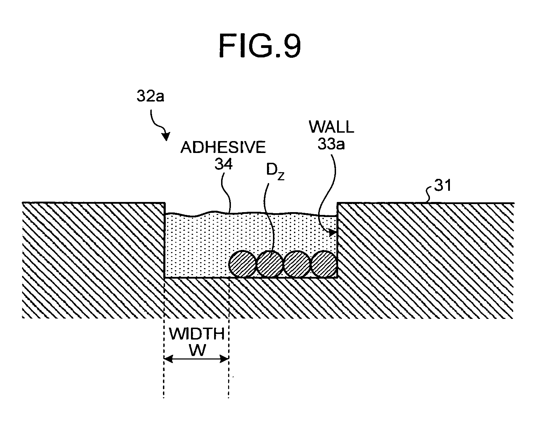

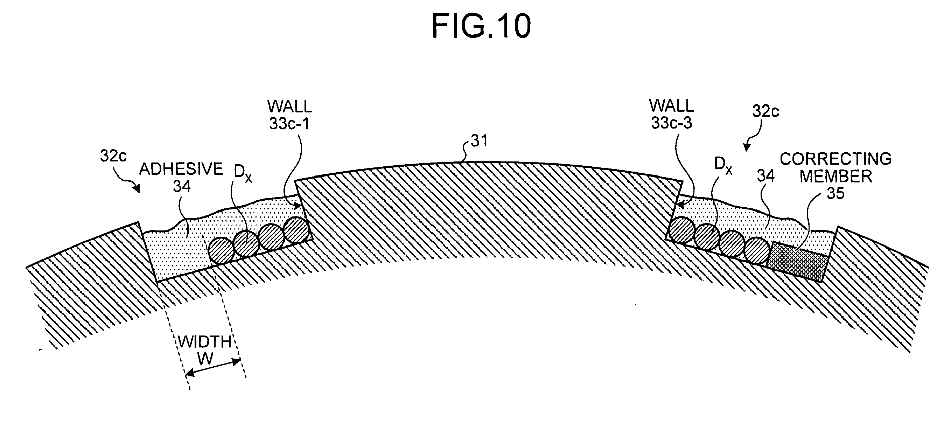

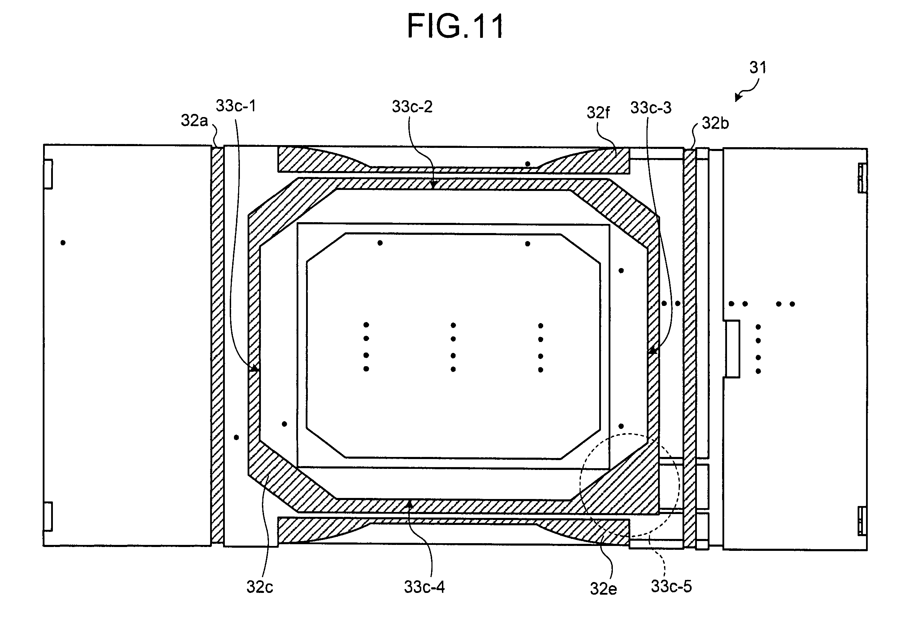

The magnetic field generator 12 including the plural sets of the drive coils D_{X}, D_{Y}, and D_{Z} is explained in detail next. FIG. 8 is a schematic perspective view of a configuration example of the magnetic field generator 12 including plural sets of the drive coils D_{X}, D_{Y}, and D_{Z}. FIG. 9 is a cross-sectional schematic diagram of a fitting part of the drive coils of a longitudinal cross section of the cylindrical magnetic field generator 12. FIG. 10 is a cross-sectional schematic diagram of the fitting part of the drive coils of a vertical cross section of the cylindrical magnetic field generator 12. FIG. 11 is a schematic diagram of a configuration example of a cylindrical member for fitting plural sets of the drive coils D_{X}, D_{Y}, and D_{Z}.

As shown in FIG. 8, the cylindrical magnetic field generator 12 includes the plural sets of the drive coils D_{X}, D_{Y}, and D_{Z} and a cylindrical member 31 for fitting the plural sets of the drive coils D_{X}, D_{Y}, and D_{Z} to an outer circumference thereof. The plural sets of the drive coils D_{X}, D_{Y}, and D_{Z} are respectively fitted to respective grooves formed on an external surface of the cylindrical member 31, so that the plural sets of the drive coils D_{X}, D_{Y}, and D_{Z} are arranged in a mode of a Helmholtz coil (that is, a mode in which the magnetic field in the X-axis direction, the magnetic field in the Y-axis direction, and the magnetic field in the Z-axis direction of the absolute coordinate system can be respectively formed).

The cylindrical member 31 is a member having a cylindrical shape formed of a nonconductive and nonmagnetic resin or the like. As shown in FIGS. 8 and 11, a plurality of grooves 32a, 32b, 32c, 32d, 32e, and 32f for fitting plural sets of the drive coils D_{X}, D_{Y}, and D_{Z} are formed on the external surface of the cylindrical member 31. A longitudinal central axis of the cylindrical member 31 matches the Z-axis of the absolute coordinate system, and two axes orthogonal to each other in the circumferential direction of the cylindrical member 31 match the X-axis and Z-axis of the absolute coordinate system.

The grooves 32a and 32b arrange a set of the drive coils D_{Z} that releases the magnetic field in the Z-axis direction of the absolute coordinate system, and are arranged symmetrical to a circumferential axis of the cylindrical member 31. Specifically, the groove 32a is formed in a groove structure continuous over an entire circumference of the cylindrical member 31. As shown in FIG. 9, the groove 32a has a wide width as compared with the drive coil D_{Z} and a sufficient depth (for example, about 3 millimeters) for burying the drive coil D_{Z} by an adhesive 34. The width of the groove 32a includes a relief part having the width W for easily accommodating the drive coil D_{Z}. A wall 33a of the groove 32a is formed vertical to the Z-axis and substantially flat, and functions as a positioning unit that determines the position of the drive coil D_{Z} with respect to the cylindrical member 31. That is, the drive coil D_{Z} accommodated in the groove 32a is fitted to the cylindrical member 31 in a state abutted against the wall 33a of the groove 32a, and is buried by the adhesive 34. As a result, the drive coil D_{Z} is arranged and fixed at a specific position (position provided by the wall 33a) in the cylindrical member 31. The other groove 32b is formed in a groove structure symmetrical to the groove 32a with respect to the circumferential axis of the cylindrical member 31, and the drive coil D_{Z} is arranged and fixed therein in the same manner as the groove 32a.

The grooves 32c and 32d arrange a set of the drive coils D_{X} forming the magnetic field in the X-axis direction of the absolute coordinate system, and are formed symmetrical to the longitudinal central axis of the cylindrical member 31 in a range on the external surface of the cylindrical member and surrounded by the pair of grooves 32a and 32b. Specifically, as shown in FIG. 11, the groove 32c is formed in a substantially square shape surrounding the circumferential axis of the cylindrical member 31 matching the X-axis of the absolute coordinate system at a center. Inner walls 33c-1, 33c-2, 33c-3, and 33c-4 of the respective sides of the substantially square groove 32c are formed substantially flat, and a wire drawing part 33c-5 for drawing a wire of the drive coil is formed in one of four corners of the groove 32c. The walls 33c-1 and 33c-2 connected to a corner of the groove 32c facing the wire drawing part 33c-5, of the walls 33c-1, 33c-2, 33c-3, and 33c-4 of the groove 32c, function as a positioning part for determining the position of the drive coil D_{X} with respect to the cylindrical member 31. Bottoms of the four corners of the groove 32c and the bottom near the walls 33c-1 and 33c-3 are curved in a circular arc along the external shape of the cylindrical member 31.

As shown in FIG. 10, the groove 32c has a wide width as compared with the drive coil D_{X} and a sufficient depth (for example, about 3 millimeters) for burying the drive coil D_{X} by the adhesive 34. The width of the groove 32c includes a relief part having the width W for easily accommodating the drive coil D_{X}.

The drive coil D_{X} accommodated in the groove 32c is buried by the adhesive 34 in a state abutted against the walls 33c-1 and 33c-2 of the groove 32c. As a result, the drive coil D_{X} is arranged and fixed at a specific position (a position provided by the walls 33c-1 and 33c-2) in the cylindrical member 31. When the drive coil D_{X} is curved in the walls 33c-3 and 33c-4 facing the walls 33c-1 and 33c-2, a correcting member 35 that corrects a curved shape of the drive coil D_{X} to a linear shape is inserted into the groove 32c as required (see FIG. 10). In this case, the correcting member 35 can maintain a state that the drive coil D_{X} is abutted against the walls 33c-3 and 33c-4 of the groove 32c.

The other groove 32d (see FIG. 8) for arranging the drive coil D_{X} is formed in the same groove structure as the groove 32c in a mode facing the groove 32c, putting the longitudinal central axis of the cylindrical member 31 therebetween, and the drive coil D_{X} is arranged and fixed in the same manner as the groove 32c.

The grooves 32e and 32f arrange a set of the drive coils D_{Y} releasing the magnetic field in the Y-axis direction of the absolute coordinate system, and are formed symmetrical to the longitudinal central axis of the cylindrical member 31 in a range on the external surface of the cylindrical member and surrounded by the pair of grooves 32a and 32b. The respective grooves 32e and 32f are formed in a substantially square shape surrounding the circumferential axis of the cylindrical member 31 matching the Y-axis of the absolute coordinate system at the center. The groove structure of the grooves 32e and 32f are the same as those of the grooves 32c and 32d.

Bottoms of the grooves 32c, 32d, 32e, and 32f in a substantially square shape are curved in a circular arc along the external shape (cylindrical shape) of the cylindrical member 31. On the other hand, the respective drive coils D_{X} and D_{Y} fitted to the grooves 32c, 32d, 32e, and 32f are elastic members in a substantially flat annular shape. Accordingly, when the respective drive coils D_{X} and D_{Y} are fixed in the grooves 32c, 32d, 32e, and 32f, a distortion occurs in the drive coils D_{X} and D_{Y} due to a residual stress at the time of molding. As a result, the drive coils D_{X} and D_{Y} are curved inside the respective grooves 32c, 32d, 32e, and 32f, and thus highly accurate positioning of the drive coils D_{X} and D_{Y} with respect to the cylindrical member 31 becomes difficult and fitting of the drive coils D_{X} and D_{Y} to the cylindrical member 31 becomes difficult. To avoid such a problem, jigs and tools (hereinafter, "holding jig") for respectively holding the respective drive coils D_{X} and D_{Y} in the grooves 32c, 32d, 32e, and 32f are used to fix the respective drive coils D_{X} and D_{Y} to the cylindrical member 31.

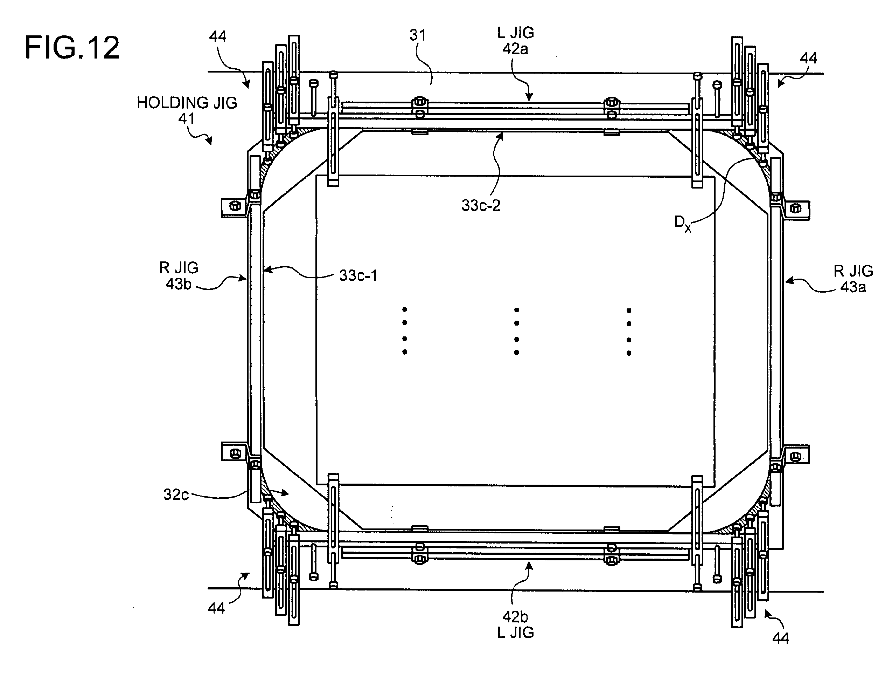

The holding jig for holding the drive coils in the groves of the cylindrical member 31 is explained next by exemplifying a case that the drive coil D_{X} is held in the substantially square groove 32c. FIG. 12 is a schematic diagram of a configuration example of the holding jig for holding the drive coil in the groove of the cylindrical member 31. As shown in FIG. 12, a holding jig 41 is arranged along the substantially square groove 32c formed on the external surface of the cylindrical member 31, and is fitted to the external surface of the cylindrical member 31 by screwing or the like. In this state, the holding jig 41 holds down the drive coil D_{X} in a range of a predetermined depth from the bottom of the groove 32c. The drive coil D_{X} held in the groove 32c by the holding jig 41 is abutted against the walls 33c-1 and 33c-2 of the groove 32c to be arranged at a specific position in the cylindrical member 31.

Specifically, the holding jig 41 includes a pair of L jigs 42a and 42b and a pair of R jigs 43a and 43b. The pair of L jigs 42a and 42b hold the side of the drive coil D_{X} along a side parallel to the longitudinal axis of the cylindrical member 31 (for example, a linear side exemplified by a side corresponding to the wall 33c-2) of the respective sides of the groove 32c. The pair of L jigs 42a and 42b has a corner holding jig 44 at the opposite ends. The corner holding jig 44 holds down the corner of the drive coil D_{X} along the corner of the groove 32c.

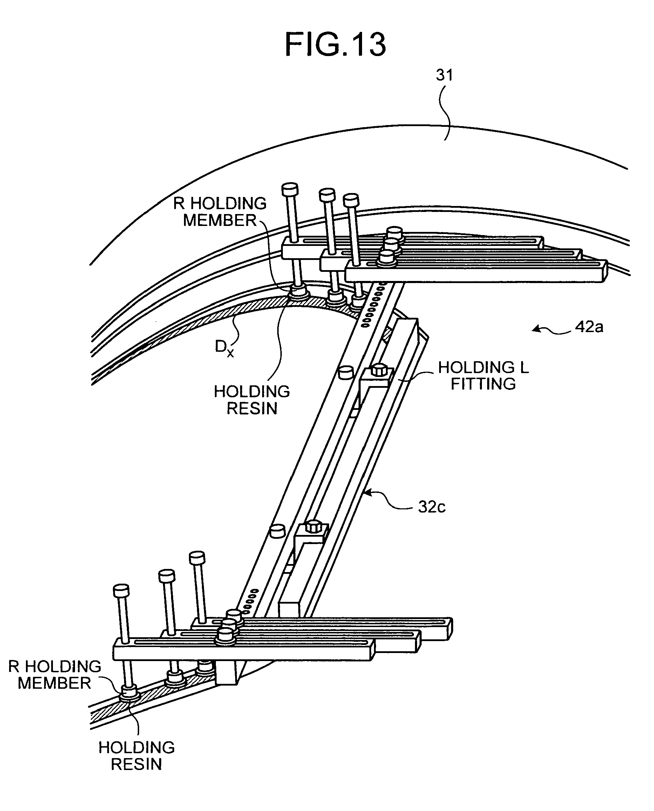

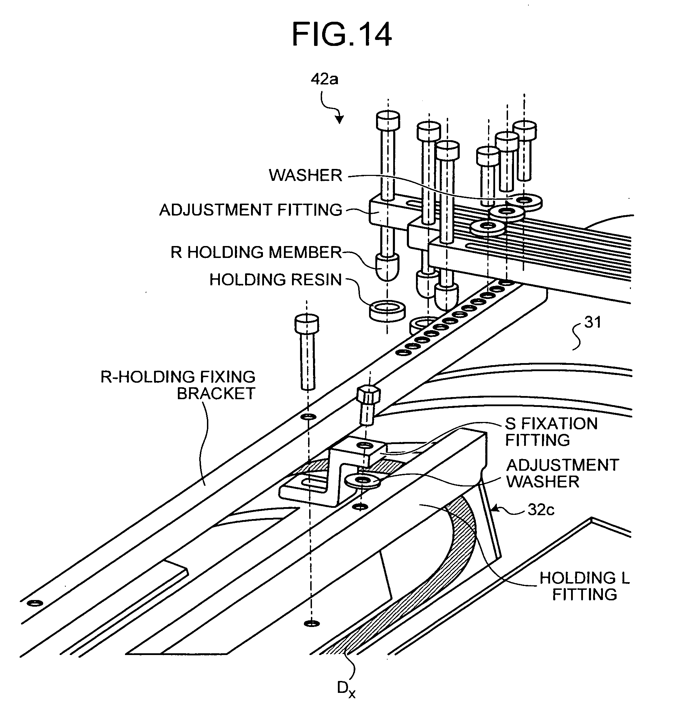

FIG. 13 is a schematic diagram of a configuration example of the L jig 42a, which is a part of the holding jig 41. FIG. 14 is a schematic diagram of a state for fitting the L jig 42a to the cylindrical member 31. As shown in FIGS. 13 and 14, the L jig 42a is realized by combining an R-holding fixing bracket as a main spindle, a holding L fitting that holds down the side of the drive coil D_{X}, an R holding member and a holding resin for holding the corner of the drive coil D_{X}, an adjustment fitting that adjusts the position of the R holding member, and predetermined fixing parts (S fixation fitting, adjustment washer, screw and the like). In this case, the holding L fitting is screwed to the R-holding fixing bracket by using the S fixation fitting, the adjustment washer and the like. The adjustment fitted with the R holding member is screwed to the R-holding fixing bracket by using a washer or the like. The R holding member, the adjustment fitting and the like fixed to the R-holding fixing bracket form the corner holding jig 44. It is desired that the holding L fitting and the holding resin coming in contact with the drive coil is manufactured by adhering an adhesion retardant tape (for example, seal of Teflon^{®}) to a contact surface with the drive coil, or by manufacturing at least the contact surface with the drive coil thereof by using an adhesion retardant material.

The L jig 42a having such a configuration is screwed to a predetermined position on the external surface of the cylindrical member 31. The holding L fitting of the L jig 42a in a screwed state holds down the side of the drive coil D_{X} along the linear side of the groove 32c. As a result, the L jig 42a holds down the side of the drive coil D_{X} in the range of a predetermined depth from the bottom of the groove 32c. The R holding member of the L jig 42a in the screwed state holds down the corner of the drive coil D_{X} along the corner of the groove 32c via the holding resin. As a result, the L jig 42a holds down the corner of the drive coil D_{X} in the range of the predetermined depth from the bottom of the groove 32c. The L jig 42b forming a pair with the L jig 42a has the same structure and function as those of the L jig 42a.

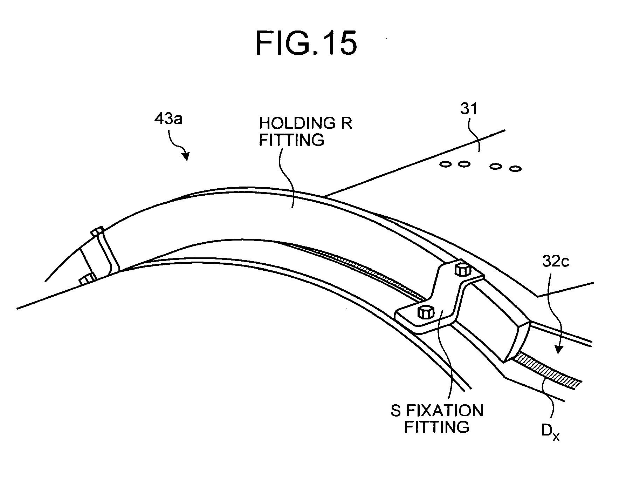

On the other hand, as shown in FIG. 12, a pair of the R jigs 43a and 43b holds down the side of the drive coil D_{X} along the side (for example, the side having a circular-arc shape exemplified by the side corresponding to the wall 33c-1) parallel to the circumferential direction of the cylindrical member 31, of the respective sides of the groove 32c. FIG. 15 is a schematic diagram of a configuration example of the R jig 43a, which is a part of the holding jig 41. As shown in FIG. 15, the R jig 43a is realized by combining a circular-arc holding R fitting that holds down the side of the drive coil D_{X}, the S fixation fitting, and the fixing part such as a screw. In this case, the holding R fitting is fixed to the external surface of the cylindrical member 31 by using the S fixation fitting and the screw.

The R jig 43a having such a configuration is screwed to the external surface of the cylindrical member 31 along the circular-arc side of the groove 32c, and the holding R fitting of the R jig 43a in a screwed state holds down the side of the drive coil D_{X} along the circular-arc side of the groove 32c. As a result, the R jig 43a maintains the side of the drive coil D_{X} in the circular-arc shape along the bottom of the groove 32c, and holds down the side of the drive coil D_{X} in the range of the predetermined depth from the bottom of the groove 32c. The R jig 43b forming a pair with the R jig 43a has the same structure and function as those of the R jig 43a.

The holding jig 41 including the L jigs 42a and 42b and the R jigs 43a and 43b can be used in the same manner as in the groove 32c, at the time of holding down the drive coils D_{X} and D_{Y} in the remaining substantially square grooves 32d, 32e, and 32f.

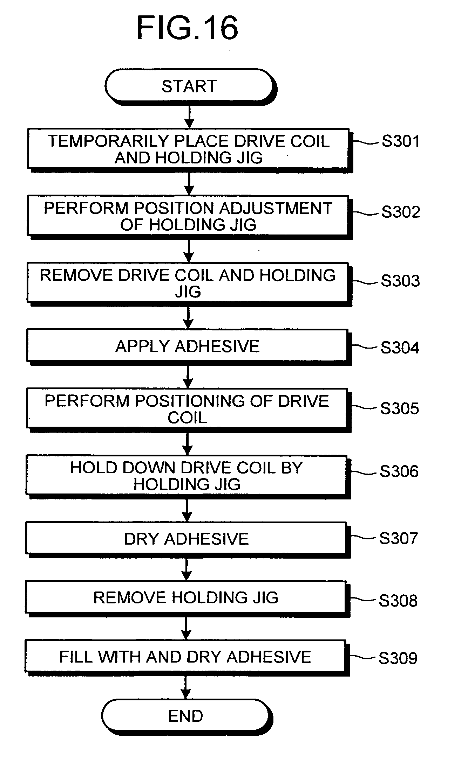

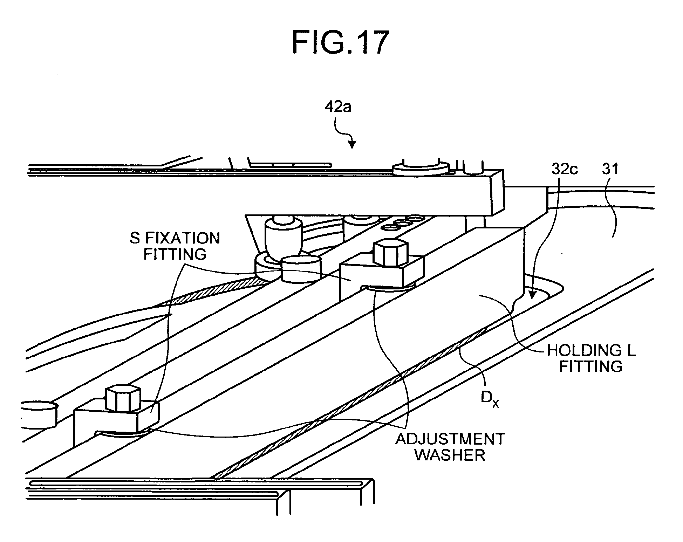

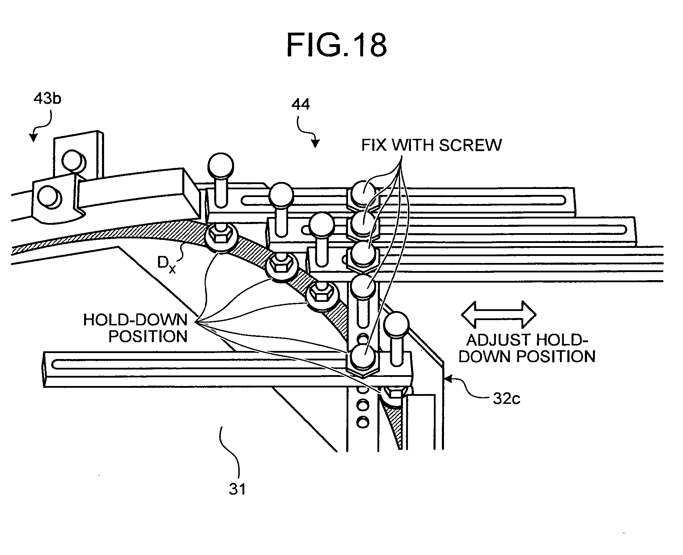

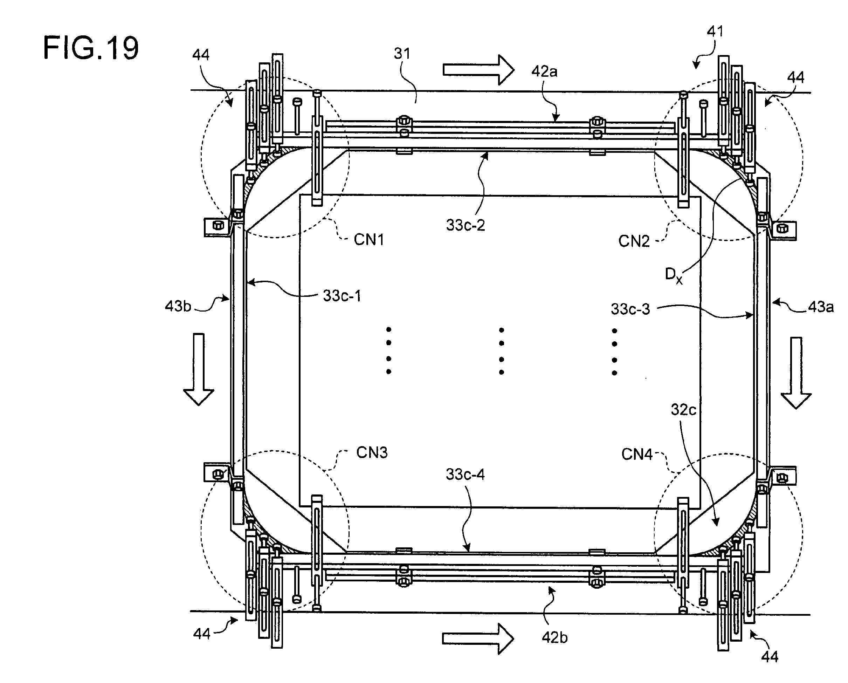

An operation procedure when the drive coil is fixed in the groove of the cylindrical member 31 is explained next, by exemplifying a case that the drive coil D_{X} is fixed in the substantially square groove 32c. FIG. 16 is a flowchart for exemplifying the operation procedure for arranging and fixing the drive coil in the groove of the cylindrical member 31. FIG. 17 is a schematic diagram of position adjustment of the holding jig with respect to the side of the drive coil. FIG. 18 is a schematic diagram of position adjustment of the holding jig with respect to the corner of the drive coil. FIG. 19 is a schematic diagram of explaining a procedure for holding down the drive coil in the groove by the holding jig.

As shown in FIG. 16, the drive coil D_{X} to be fitted and the holding jig 41 are temporarily placed on the external surface of the cylindrical member 31 (Step S301). At Step S301, the drive coil D_{X} is arranged in the groove 32c of the cylindrical member 31 and is abutted against the walls 33c-1 and 33c-2 of the groove 32c. Accordingly, positioning of the drive coil D_{X} with respect to the cylindrical member 31 is temporarily performed. The L jigs 42a and 42b and the R jigs 43a and 43b are screwed along the groove 32c (see FIGS. 14 and 15), so that the holding jig 41 is temporarily placed on the external surface of the cylindrical member 31.

Position adjustment of the holding jig 41 is then performed by using the drive coil D_{X} temporarily placed inside the groove 32c (Step S302). At Step S302, the position of the holding jig 41 is adjusted so that the holding jig 41 holds down the drive coil D_{X} temporarily positioned with respect to the cylindrical member 31. Specifically, as shown in FIG. 17, the position of the L jig 42a of the holding jig 41 is adjusted so that the holding L fitting holds down the side of the drive coil D_{X} in the groove 32c by adjusting the S fixation fitting. As shown in FIG. 18, the position of the corner holding jig 41 of the L jig 42a is adjusted so that the R holding member and the holding resin hold down the corner of the drive coil D_{X} in the groove 32c by using the adjustment fitting. The position of the L jig 42b forming a pair with the L jig 42a is adjusted in the same manner as in the L jig 42a. On the other hand, as shown in FIGS. 15 and 18, the positions of the R jigs 43a and 43b of the holding jig 41 are adjusted so that the R jigs 43a and 43b hold down the side of the drive coil D_{X} to the circular-arc side to the groove 32c.

Subsequently, the drive coil D_{X} and the holding jig 41 temporarily placed on the external surface of the cylindrical member 31 are removed (Step S303), and the adhesive 34 for adhering the drive coil D_{X} is applied to the groove 32c of the cylindrical member 31 (Step S304). At Step S303, the holding jig 41 is removed from the external surface of the cylindrical member 31, while maintaining a state position-adjusted at Step S302. At Step S304, the adhesive 34 is applied to the contact surface between the bottom of the groove 32c and the drive coil D_{X} to penetrate sufficiently. The adhesive 34 is not for adhering the drive coil momentarily but for adhering the drive coil in such a manner that the adhesive does not become solidified until a predetermined time (for example, a sufficient time for holding the drive coil D_{X} by the holding jig 41 while positioning the drive coil D_{X} with respect to the cylindrical member 31) passes, and adheres the drive coil in the groove after the predetermined time has passed.

The drive coil D_{X} is then arranged again in the groove 32c applied with the adhesive 34, thereby performing positioning of the drive coil D_{X} with respect to the cylindrical member 31 (Step S305). At Step S305, the drive coil D_{X} is abutted against the walls 33c-1 and 33c-2 of the groove 32c that function as the positioning part, thereby achieving positioning of the drive coil D_{X} with respect to the cylindrical member 31.

Thereafter, the holding jig 41 is fitted again to the external surface of the cylindrical member 31 along the groove 32c in which the drive coil D_{X} is positioned with respect to the cylindrical member 31, and the drive coil D_{X} is held down in the groove 32c by the fitted holding jig 41 (Step S306). At Step S306, as shown in FIG. 19, the holding jig 41 is fitted to the external surface of the cylindrical member 31, matched with the position of the drive coil D_{X} in the groove 32c, and the respective sides and corners of the drive coil D_{X} are held in a predetermined sequence to fix the position of the drive coil D_{X}.

Specifically, the holding jig 41 holds down a corner CN1 of the drive coil D_{X} to the corner of the groove 32c formed by the walls 33c-1 and 33c-2 crossing each other, against which the drive coil D_{X} is abutted (that is, the corner facing the wire drawing part 33c-5). The holding jig 41 then sequentially holds down the respective sides and corners of the drive coil D_{X} along a direction shown by a thick arrow in FIG. 19. In this case, the holding jig 41 holds the side of the drive coil D_{X} abutted against the wall 33c-2 by the L jig 42a, and the side of the drive coil D_{X} abutted against the wall 33c-1 by the R jig 43b. Subsequently, the holding jig 41 holds down a corner CN2 of the drive coil D_{X} to a corner of the groove 32c formed by the walls 33c-2 and 33c-3 crossing each other by the corner holding jig 44, and holds down a corner CN3 of the drive coil to a corner of the groove 32c formed by the walls 33c-1 and 33c-4 crossing each other by the corner holding jig 44. Thereafter, the holding jig 41 holds down the side of the drive coil D_{X} arranged near the wall 33c-4 by the L jig 42b, and holds down the side of the drive coil D_{X} arranged near the wall 33c-3 by the R jig 43a. Last, the holding jig 41 holds down a corner CN4 of the drive coil D_{X} to a corner of the groove 32c formed by the walls 33c-3 and 33c-4 crossing each other (that is, the corner that functions as the wire drawing part 33c-5) by the corner holding jig 44.

After the holding jig 41 holds down the respective sides and corners of the drive coil D_{X} in the groove 32c, the adhesive between the drive coil D_{X} and the bottom of the groove 32c, that is, the adhesive 34 applied at Step S304 is dried (Step S307) to adhere the drive coil Dx to the groove 32c.

Next, after the holding jig 41 is removed from the external surface of the cylindrical member 31 (Step S308), the adhesive 34 is filled in the groove 32c to which the drive coil D_{X} is bonded, and the filled adhesive 34 is dried (Step S309) to finish the operation. As a result, the drive coil D_{X} arranged at a specific position with respect to the cylindrical member 31 is completely fixed to the groove 32c. At Step S309, the adhesive 34 is filled in the groove so as not to overflow from the groove accommodating the drive coil as shown in FIGS. 9 and 10 (for example, up to a depth of 2 millimeters with respect to the groove having a depth of 3 millimeters).

When the holding jig 41 holds down the drive coil D_{X} in the groove 32c (Step S306) as described above, the holding jig 41 holds down the substantially flat drive coil D_{X} to the bottom of the groove 32c curved in the circular-arc shape along the external shape of the cylindrical member 31. Therefore, a curved portion distorted with respect to the groove 32c can be generated in a part of the respective sides of the drive coil D_{X} (specifically, the side of the drive coil D_{X} positioned near the wall 33c-3 or 33c-4). When the curved portion is generated in the drive coil D_{X}, it is difficult to position the drive coil D_{X} with respect to the cylindrical member 31 highly accurately.

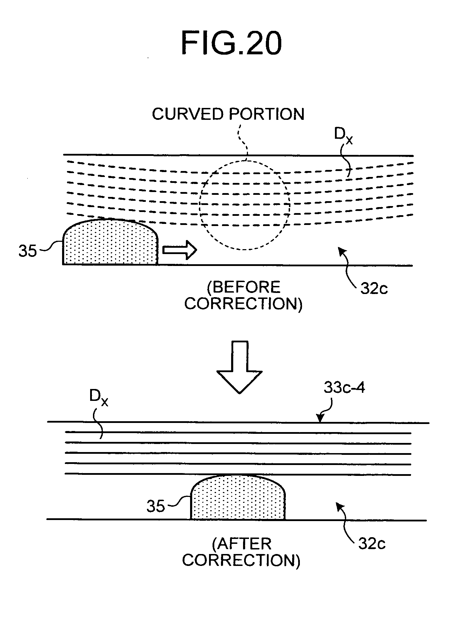

To solve such a problem, the correcting member 35 can be inserted, as required, into the groove 32c in which the drive coil D_{X} is arranged, so that the curved portion of the drive coil D_{X} is corrected to a linear shape by the correcting member 35. FIG. 20 is a schematic diagram of a state that the curved portion of the drive coil D_{X} is corrected to a linear shape by the correcting member 35.

The correcting member 35 is inserted into the groove 32c, for example, at Step S305 or S306. As shown in FIG. 20, the correcting member 35 inserted into the groove 32c slides along the wall of the groove 32c to reach the curved portion of the drive coil D_{X} and presses the curved portion against the wall (for example, the wall 33c-4) of the groove 32c, thereby correcting the curved portion to a linear shape. The correcting member 35 can maintain the shape of respective sides of the drive coil D_{X} arranged in the groove 32c in the linear shape, and can abut the respective sides of the drive coil D_{X} against not only the walls 33c-1 and 33c-2 that function as the positioning part but also the remaining walls 33c-3 and 33c-4. Positioning of the drive coil D_{X} can be performed by abutting the respective sides of the drive coil D_{X} against the four walls 33c-1, 33c-2, 33c-3, and 33c-4 of the grooves 32c due to the action of the correcting member 35, and as a result, the positioning accuracy of the drive coil D_{X} with respect to the cylindrical member 31 can be increased.

Fixation of the drive coils D_{X} and D_{Y} to the remaining substantially square grooves 32d, 32e, and 32f is achieved by repeating steps S301 to S309. In this case, operational effects of the correcting member 35 same as those for the drive coil D_{X} described above can be acquired for the drive coils D_{X} and D_{Y} in the respective grooves 32d, 32e, and 32f by inserting the correcting member 35 into the grooves 32d, 32e, and 32f as required.

As explained above, in the embodiment of the present invention, the switching unit switches the drive coil in an active state that applies the magnetic field to the LC marker contained in a detected object such as a capsule endoscope from the drive coils. The control unit determines the selecting condition of the drive coil based on predetermined information stored in the storage unit in advance (for example, the magnetic-field information table 19a and the hysteresis coefficient A less than 1) to select a drive coil that satisfies the determined selecting condition from the drive coils, and controls the drive-coil switching operation of the switching unit so that the selected drive coil is switched to an active state. Further, the control unit detects the induced magnetic field generated from the LC marker in the detected object by the magnetic field of the selected drive coil in an active state by a plurality of sense coils, thereby detecting the position information of the detected object based on the detection result of the field strength of the induced magnetic field acquired by the sense coils. Accordingly, a most appropriate drive coil (for example, the drive coil having the largest strength of a magnetic field component that generates the induced magnetic field to the LC marker) can be selected from the drive coils according to the position and direction of the LC marker in the detected object that is displaced or changes the orientation in a predetermined three-dimensional space, and the drive coil in an active state can be hysteretically switched based on the selecting condition of the drive coil determined by using the hysteresis coefficient. Accordingly, it is possible to avoid a case such that the drive coil in an active state is repeatedly switched within a short time according to the position of the detected object, every time the detected object is displaced in the three-dimensional space. As a result, the transient characteristic and the temperature characteristic after the drive coil is switched can be stabilized, and the stable magnetic field can be applied to the LC marker, thereby enabling to realize a position detecting device that can stably perform a position detecting process of the detected object incorporating the LC marker therein.

The inappropriate sense coil, in which the strength detection value of the magnetic field of the drive coil in an active state is saturated or substantially zero, of the sense coils that detects the induced magnetic field generated from the LC marker in the detected object due to the magnetic field of the drive coil currently in an active state, is invalidated to select respective remaining sense coils, and the position information of the detected object is calculated based on the detection result of the field strength of the induced magnetic field acquired by the selected sense coils. Accordingly, the position information of the detected object can be calculated by using only the detection value having high reliability from the detection values of the induced magnetic field acquired by the sense coils. As a result, a position detecting device that can increase the accuracy of the position detection of the detected object incorporating the LC marker therein and can perform a stable position detecting process of the detected object can be realized.

Furthermore, at the time of switching the drive coil in an active state among the drive coils, the relay corresponding to the drive coil in an active state is switched off after a certain time has passed since suspension of an output of the alternating-current signal to be applied to the drive coil in an active state, and the relay corresponding to another drive coil is switched on after a certain time has passed since switching off of the relay corresponding to the drive coil in an active state. The FFT processing with respect to the detection result of the field strength of the induced magnetic field is then stopped in a switching period of the drive coil in an active state. Accordingly, chattering of the relay at the time of switching of the drive coil and an unnecessary residual current can be eliminated, and an adverse effect to the FFT processing (such as the execution of incorrect FFT processing) due to chattering of the relay or the residual current can be prevented. As a result, the accuracy of the position detection of the detected object at the time of switching the drive coil in an active state among the drive coils can be increased.

When a plurality of drive coils are fixed to the external surface of the cylindrical member, for which the absolute coordinate is specified, to constitute plural sets of drive coils arranged in a mode of a Helmholtz coil, the drive coils, which are substantially flat elastic members, are arranged and fixed to the external surface of the cylindrical member by using a predetermined holding jig that holds down the respective drive coils, matched with the circular-arc shape of the cylindrical member. Accordingly, distortion of the drive coils (curved deformation) due to a residual stress generated at the time of fixing the substantially flat drive coils to the external surface of the cylindrical member having the circular-arc shape can be suppressed. As a result, the plural sets of drive coils can be positioned with respect to the cylindrical member highly accurately, and the plural sets of drive coils can be easily arranged and fixed to the external surface of the cylindrical member.

In the embodiment of the present invention described above, the sense coil selector 20b in the control unit 20 selects a sense coil effective for the position information calculating process based on the calibration value of the respective sense coils 16c. However, the present invention is not limited thereto, and a sense coil selector that selects a sense coil according to the drive coils D_{X}, D_{Y}, and D_{Z} that apply the magnetic field to the LC marker 2a can be provided.

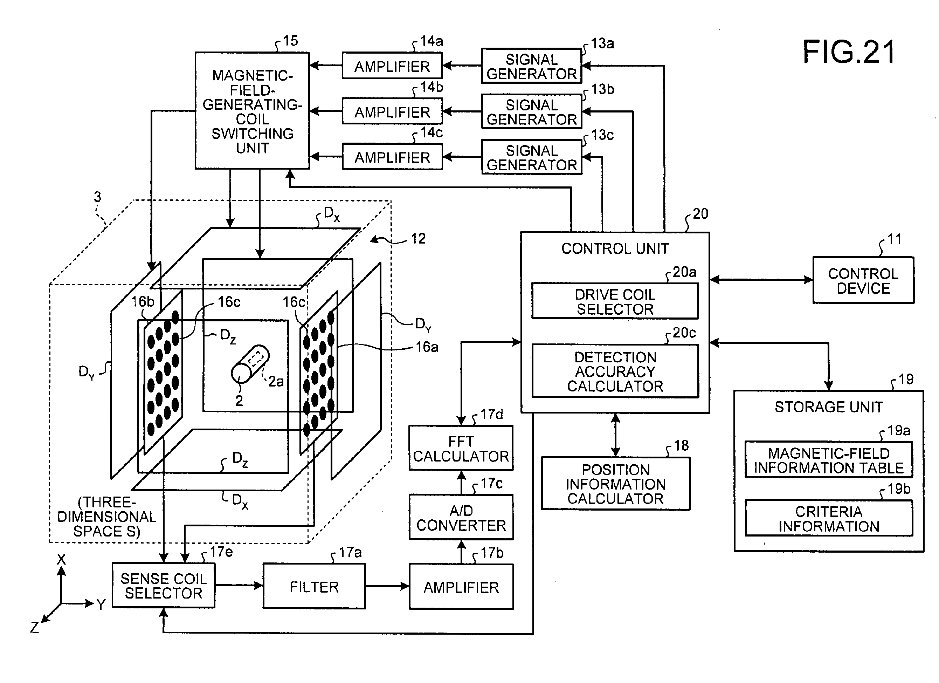

Specifically, as shown in FIG. 21, a sense coil selector 17e acquires the detection result of the field strength of the induced magnetic field from the respective sense coils 16c in the magnetic field detectors 16a and 16b, to select the respective sense coils instructed to be selected by the control unit 20 corresponding to the drive coils (any one of plural sets of the drive coils D_{X}, D_{Y}, and D_{Z}) in an active state from the acquired detection result of the field strength, and transmits the detection result of the field strength acquired by the selected respective sense coils to the filter 17a. In this case, the control unit 20 can set a sense coil to be selected for each drive coil in advance, and put the drive coil in an active state and the respective sense coils that detect the induced magnetic field of the LC marker 2a in one-to-one correspondence with each other, so that the respective sense coils corresponding to the drive coils in an active state are selected (switched) by the sense coil selector 17e. A position detecting device according to a modification of the embodiment of the present invention shown in FIG. 21 does not include the sense coil selector 20b in the position detecting device 10 according to the embodiment described above, but includes the sense coil selector 17e instead thereof. In this case, the control unit 20 controls the operation of the sense coil selector 17e. Other parts of the configuration are the same as those in the above embodiment, and like reference letters or numerals are denoted to like components.

In the embodiment of the present invention described above, at the time of switching the drive coils in an active state of plural sets of the drive coils D_{X}, D_{Y}, and D_{Z}, for example, as shown in FIG. 5, an output of the alternating-current signal to the drive coils D_{X} in an active state and the relay are switched off, and then the alternating-current signal to other drive coils D_{Y} is switched on. However, the present invention is not limited thereto, and the alternating-current signal to the drive coils in a non-active state and the relay can be switched on, and thereafter, the output of the alternating-current signal to the drive coils currently in an active state and the relay can be switched off.

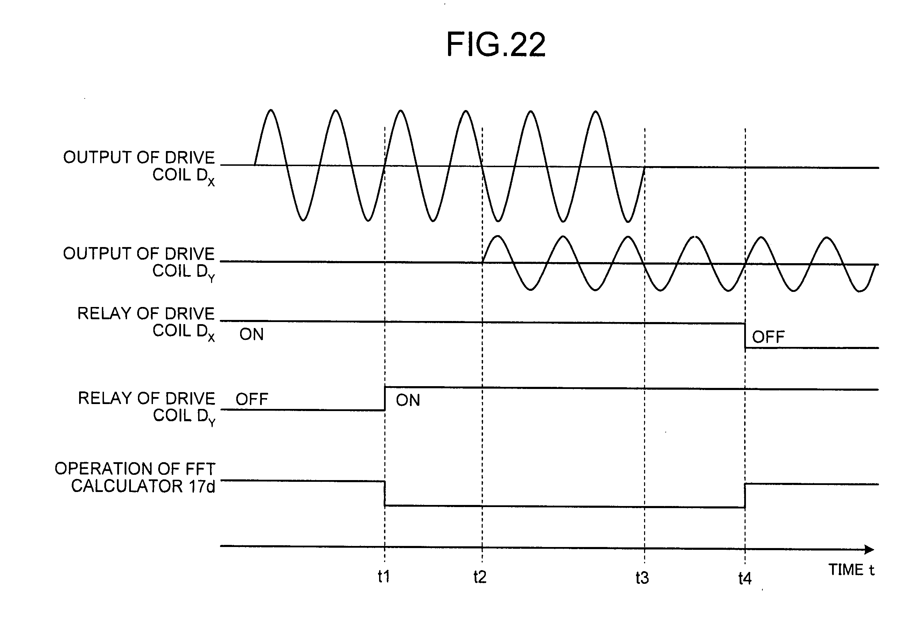

Specifically, when an active state is switched from the drive coils D_{X} currently in an active state to other drive coils D_{Y}, as shown in FIG. 22, the control unit 20 controls the magnetic-field-generating-coil switching unit 15 to switch the relay of the drive coils D_{X} to be on at a timing (a time t1) at which the FFT calculator 17d completes the execution of the FFT processing for one section, and controls the signal generator 13b at a timing (a time t2) after a certain time has passed since the time t1, thereby starting to output the alternating-current signal to the drive coils D_{Y} in a non-active state. In this case, the control unit 20 causes the signal generator 13b to start the output of the alternating-current signal after the amplitude of the alternating-current signal (sine wave) to the drive coils D_{Y} becomes substantially zero.
The control unit 20 controls the signal generator 13a at a timing (a time t3) after a certain time has passed since the time t2, to stop the output of the alternating-current signal to the drive coils D_{X} in an active state. In this case, the control unit 20 causes the signal generator 13a to stop the output of the alternating-current signal after the amplitude of the alternating-current signal (sine wave) to the drive coils D_{X} becomes substantially zero. Subsequently, the control unit 20 controls the magnetic-field-generating-coil switching unit 15 to switch the relay of the drive coils D_{X} to be off at a timing (a time t4) after a certain time has passed since the time t3. Thereafter, the control unit 20 then causes the FFT calculator 17d to restart the FFT processing at a timing of the time t4.

As shown in FIG. 22, operational effect as those of the above embodiment can be achieved by performing the switching control of the drive coils in an active state by the control unit 20, and a rise time (that is, a time required since starting to apply the alternating-current signal until stable amplitude and alternating field of the frequency can be emitted) of the drive coil newly switched to an active state can be sufficiently ensured. As a result, the transient characteristic of the drive coil at the time of switching the drive coil in an active state among the drive coils can be stabilized further, and the position detection accuracy of the detected object at the time of switching the drive coil in an active state can be increased further.

In the embodiment of the present invention described above, the grooves 32a, 32b, 32c, 32d, 32e, and 32f for arranging and fixing the respective sets of the drive coils D_{X}, D_{Y}, and D_{Z} are formed on the external surface of the cylindrical member 31 in a mode having the substantially rectangular cross section, however, an undercut for the adhesive 34 can be provided on the bottoms of the grooves 32a, 32b, 32c, 32d, 32e, and 32f.

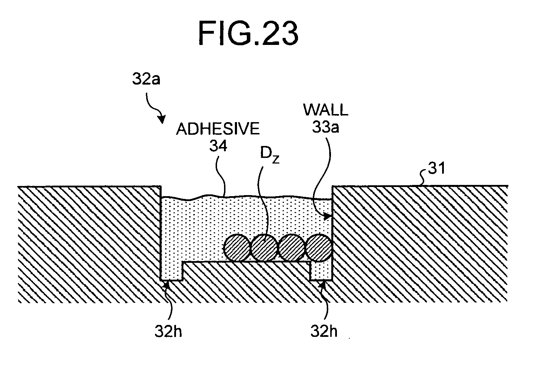

Specifically, as shown in FIG. 23, an undercut 32h of a desired depth can be formed along the groove 32a near the wall of the bottom of the groove 32a in which the drive coil D_{Z} is arranged and fixed. The undercut 32h is a groove for allowing the adhesive 34 applied or filled in the groove 32a to flow. It is possible to prevent a case such that the adhesive 34 overflows outside of the groove 32a due to the action of the undercut 32h, and interposition of the adhesive 34 between the wall 33a and the drive coil D_{Z} can be suppressed. As a result, positioning of the drive coil D_{Z} with respect to the cylindrical member 31 can be reliably realized, while preventing wasteful leakage of the adhesive 34 on the external surface of the cylindrical member 31. The undercut 32h can be formed also on the respective bottoms of other grooves 32b, 32c, 32d, 32e, and 32f, and same operational effect of the undercut 32h as those in the case of groove 32a can be acquired. As a result, positioning of the plural sets of the drive coils D_{X}, D_{Y}, and D_{Z} with respect to the cylindrical member 31 can be reliably realized, while preventing wasteful leakage of the adhesive 34 on the external surface of the cylindrical member 31.

In the embodiment of the present invention described above, the respective thresholds, which are values acquired by multiplying the respective field strengths Eₙₚ₁ and Enₙₚ₂ (any two of the field strengths E_{DX}, E_{DY}, and E_{DZ}) of the drive coils in a non-active state by the preset hysteresis coefficient A, are compared with the field strength Ep (any one of the field strengths E_{DX}, E_{DY}, and E_{DZ}) of the drive coil in an active state, and drive coils in an active state are hysteretically selected (switched) from plural sets of the drive coils D_{X}, D_{Y}, and D_{Z} based on a comparison result. However, the present invention is not limited thereto, and after duration of the drive coil immediately after switching is preset instead of the hysteresis coefficient A, and the drive coil in an active state is hysteretically selected (switched) from the plural sets of the drive coils D_{X}, D_{Y}, and D_{Z}, the active state of the drive coil immediately after switched can be maintained, regardless of the field strengths E_{DX}, E_{DY}, and E_{DZ} with respect to the LC marker 2a until the set duration passes.

Specifically, the storage unit 19 stores duration T in advance, instead of the hysteresis coefficient A as the criteria information 19b (that is, a part of the information of the selecting condition of the drive coils). The control unit 20 selects the drive coil having the largest field strength of the field strengths E_{DX}, E_{DY}, and E_{DZ} with respect to the LC marker 2a, and controls the magnetic-field-generating-coil switching unit 15 to switch the selected drive coil to an active state. Thereafter, the control unit 20 maintains the drive coil currently in an active state regardless of a magnitude correlation of the field strengths E_{DX}, E_{DY}, and E_{DZ}, even if the magnitude correlation of the field strengths E_{DX}, E_{DY}, and E_{DZ} of the respective drive coils changes due to a displacement or the like of the detected object (the capsule endoscope 2) during a period since switching of the drive coil until a duration time T passes. Also in this case, operational effects as those of the above embodiment can be achieved.

In the embodiment of the present invention described above, the drive coil is initially selected by taking into consideration a coil axis direction of the LC marker 2a at the time of introducing the LC marker 2a into the three-dimensional space S. However, the present invention is not limited thereto, and a sensor that detects an initial coil axis direction of the LC marker 2a (that is, an introduction direction of the LC marker 2a) at the time of introducing the LC marker 2a (specifically, the detected object such as the capsule endoscope 2 incorporating the LC marker 2a therein) into the three-dimensional space S can be provided, and the drive coil at the time of introducing the LC marker 2a can be initially selected based on a detection result of the sensor.

Further, in the embodiment of the present invention described above, the field strengths B_{DX}, B_{DY}, and B_{DZ} to be applied to the position of the LC marker 2a by the respective drive coils is calculated by using the magnetic-field information table 19a (a look-up table indicating a correspondence between respective positions on the respective axes in the absolute coordinate system and the field strength by the drive coils). However, the present invention is not limited thereto, and a lookup table indicating a reference magnetic field, which is the field strength of the drive coil in a unitary current value (a current value of 1A) can be provided instead of the magnetic-field information table 19a, to monitor a current value (an output value of the alternating-current signal) to be applied to the respective drive coils, so that the field strengths B_{DX}, B_{DY}, and B_{DZ} can be calculated by multiplying a monitored current value by the reference magnetic field. Alternatively, coil characteristics (coil size, coil position, number of turns and the like) of the respective drive coils can be stored in advance, instead of the magnetic-field information table 19a, and the field strengths B_{DX}, B_{DY}, and B_{DZ} can be calculated based on the current value to be applied to the respective drive coils, the coil characteristics, and the position information of the detected object. As a result, a data amount to be held in the storage unit 19 can be reduced.

In the embodiment of the present invention described above, at the time of switching drive coils in an active state among plural sets of the drive coils D_{X}, D_{Y}, and D_{Z}, the FFT processing by the FFT calculator 17d is stopped. However, the present invention is not limited thereto, and the control unit 20 can allow the FFT calculator 17d to execute the FFT processing of the field strength signal even in a switching period of the drive coils, and a FFT processing result acquired by being executed in the switching period of the drive coils can be invalidated from the FFT processing results acquired by the FFT calculator 17d.

Further, in the embodiment of the present invention described above, the position detecting device 10 contained in the capsule guidance system that magnetically guides the capsule endoscope 2 introduced into the subject to detect the position information of the capsule endoscope 2 in the subject is exemplified. However, the present invention is not limited thereto, and the position detecting device according to the present invention needs only to detect the position information in the three-dimensional space of a predetermined detected object incorporating the LC marker 2a therein, and is not particularly limited to the position detecting device contained in the capsule guidance system.

The detected object whose position information is detected by the position detecting device according to the present invention needs only to incorporate the LC marker 2a therein, and is not particularly limited to a medical device. Further, the capsule medical device whose position information is detected as the detected object is not limited to the capsule endoscope 2 described above, and it can be a capsule pH measuring device that measures pH in a living body, a capsule drug-administrating device having a function of dispersing or injecting a drug into the living body, or a capsule sampling device that samples a substance in the living body.

Further, in the embodiment of the present invention described above, three sets of the drive coils D_{X}, D_{Y}, and D_{Z} are exemplified as a plurality of drive coils included in the magnetic field generator 12 that apply the magnetic field to the LC marker 2a. However, the number of drive coils included in the magnetic field generator 12 is not particularly limited to three sets (that is, six sets in total), and can be plural (seven or more, for example). In this case, a plurality of grooves are formed on the external surface of the cylindrical member corresponding to the drive coils to be arranged, and the drive coils are arranged and fixed to the respective grooves, respectively.

In the embodiment of the present invention described above, when the drive coils are sequentially switched according to a fixed sequence such as an order from the drive coils D_{X}, the drive coils D_{Y}, and the drive coils D_{Z}, the most accurate position detection result, of the respective position detection results at the time of driving the respective drive coils in the fixed sequence, can be adopted as the position detection result of the detected object. For example, at the time of performing the switching operation of the drive coils according to the fixed sequence such as the drive coils D_{X}, the drive coils D_{Y}, and the drive coils D_{Z}, if the position detection result at the time of driving the drive coils D_{X} has the highest accuracy, the position detection result at the time of driving the drive coils D_{X} is adopted as the position detection result of the detected object in a switching period of the drive coils.

### INDUSTRIAL APPLICABILITY

As described above, the position detecting device, the medical device guidance system, the position detecting method, and the medical device guiding method according to the present invention are useful to detect a position of a detected object having a resonance circuit between a coil and a capacitor contained therein, and are particularly suitable as a position detecting device, a medical device guidance system, a position detecting method, and a medical device guiding method that can stably perform a position detecting process of a detected object such as a capsule medical device.

## Claims

1. A position detecting device comprising:
a detected object that includes a circuit with at least one internal coil;
a first magnetic-field generator that includes at least a magnetic-field generating coil for generating a first magnetic field to a detection space of the detected object;
a plurality of detection coils that detect an induced magnetic field generated from the internal coil caused by the first magnetic field;
a magnetic-field-generating-coil switching unit that selects at least one magnetic-field generating coil to be operated from the magnetic-field generating coils in the first magnetic-field generator;
a storage unit that stores predetermined information for selecting at least one magnetic-field generating coil to be operated from the magnetic-field generating coils in the first magnetic-field generator; and
a control unit that controls the magnetic-field-generating-coil switching unit to select the at least one magnetic-field generating coil to be operated among the magnetic-field generating coils in the first magnetic-field generator, based on at least one of a position and a direction of the detected object and based on the predetermined information.

2. The position detecting device according to claim 1, wherein the control unit hysteretically selects the magnetic-field generating coil based on the predetermined information.

3. The position detecting device according to claim 1, wherein the predetermined information is a continuous operation time of the selected magnetic-field generating coil.

4. The position detecting device according to claim 1, wherein the predetermined information is a selection sequence of the magnetic-field generating coil.

5. The position detecting device according to claim 1, wherein the predetermined information is information of the first magnetic field generated by the magnetic-field generating coils.

6. The position detecting device according to claim 1, further comprising at least one signal generator that outputs an alternating-current signal to the at least one magnetic-field generating coil, wherein
at a time of selecting the magnetic-field generating coil, the control unit stops an output of the signal generator in an active state, and after a predetermined time has passed, disconnects between the magnetic-field generating coil which is in an active state and the magnetic-field-generating-coil switching unit, then after a predetermined time has passed, connects the selected magnetic-field generating coil with the magnetic-field-generating-coil switching unit, and then after a predetermined time has passed, starts an output of the selected signal generator.

7. The position detecting device according to claim 6, wherein the magnetic-field generating coil which is in an active state is the selected magnetic-field generating coil.

8. The position detecting device according to claim 1, further comprising at least one signal generator that outputs an alternating-current signal to the at least one magnetic-field generating coil, wherein
at a time of selecting the magnetic-field generating coil, the control unit connects the selected magnetic-field generating coil with the magnetic-field-generating-coil switching unit, and after a predetermined time has passed, starts an output of the selected signal generator, then after a predetermined time has passed, stops an output of the signal generator, which has been in an active state, and then after a predetermined time has passed, disconnects between the magnetic-field generating coil, which has been in an active state, and the magnetic-field-generating-coil switching unit.

9. The position detecting device according to claim 1, further comprising:
an A/D converter that converts a detection result of the induced magnetic field detected by the detection coils to a digital signal; and
an FFT calculator that calculates fast Fourier transform with respect to a detection result converted to the digital signal, wherein
the control unit stops an operation of at least one of the A/D converter and the FFT calculator during a period where the magnetic-field-generating-coil switching unit is selecting a magnetic-field generating coil.

10. The position detecting device according to claim 1, further comprising a position and direction calculator that calculates a position and a direction of the detected object based on detection results of the detection coils, wherein
the control unit stops calculation of a position and direction by the position and direction calculator during a period where the magnetic-field-generating-coil switching unit is selecting a magnetic-field generating coil.

11. The position detecting device according to claim 1, wherein the control unit selects an effective one among the detection coils according to the selected magnetic-field generating coil.

12. The position detecting device according to claim 1, wherein
the detection coil further detects the first magnetic field, and
the control unit selects an effective one among the detection coils according to a detection result of the first magnetic field detected by the detection coil.

13. A medical device guidance system comprising:
a medical device that includes a circuit with at least one internal coil and a magnet, the medical device being a detected object;
the position detecting device according to any one of claims 1 to 12; and
a second magnetic-field generator that generates a second magnetic field for guiding the medical device by operating the magnet.

14. A position detecting method comprising:
a first magnetic-field generating step of generating a first magnetic field to a detection space of a detected object that includes a circuit with at least one internal coil;
an induced magnetic-field detecting step of detecting, by a plurality of detection coils, an induced magnetic field generated from the internal coil caused by the first magnetic field;
a position/direction calculating step of calculating at least one of a position and a direction of the detected object based on a detection result of the induced magnetic field;
a magnetic-field generating-coil selecting step of selecting at least one magnetic-field generating coil for generating the first magnetic field, based on at least one of a position and a direction of the detected object and based on predetermined information for selecting at least one magnetic-field generating coil to be operated; and
a control step of controlling a magnetic-field-generating-coil switching unit to switch to a magnetic-field generating coil selected at the magnetic-field generating-coil selecting step.

15. The position detecting method according to claim 14, wherein the magnetic-field generating-coil selecting step includes hysteretically selecting the magnetic-field generating coil based on the predetermined information.

16. The position detecting method according to claim 14, further comprising a signal-generator selecting step of selecting at least one signal generator that outputs an alternating-current signal to the at least one magnetic-field generating coil, wherein
the control step includes:
stopping an output of the signal generator in an active state that outputs an alternating-current signal to the magnetic-field generating coil in an active state;
disconnecting, after a predetermined time has passed, a connection between the magnetic-field generating coil which is in an active state and the magnetic-field-generating-coil switching unit;
then connecting, after a predetermined time has passed, the magnetic-field generating coil selected at the magnetic-field generating-coil selecting step with the magnetic-field-generating-coil switching unit; and
then causing, after a predetermined time has passed, the signal generator selected at the signal-generator selecting step to start an output of an alternating-current signal.

17. The position detecting method according to claim 14, further comprising a signal-generator selecting step of selecting at least one signal generator that outputs an alternating-current signal to the at least one magnetic-field generating coil, wherein
the control step includes:
connecting the magnetic-field generating coil selected at the magnetic-field generating-coil selecting step with the magnetic-field-generating-coil switching unit;
starting, after a predetermined time has passed, an output of the signal generator selected at the signal-generator selecting step;
then stopping, after a predetermined time has passed, an output of the signal generator which is in an active state; and
then disconnecting, after a predetermined time has passed, between the magnetic-field generating coil which is in an active state and the magnetic-field-generating-coil switching unit.

18. The position detecting method according to claim 14, further comprising:
an A/D converting step of converting a detection result of the induced magnetic field detected by the detection coils to a digital signal by an A/D converter; and
an FFT calculating step of performing fast Fourier transform with respect to a detection result converted to the digital signal by an FFT calculator, wherein
the control step includes stopping an operation of at least one of the A/D converter and the FFT calculator during a period where the magnetic-field-generating-coil switching unit is selecting the magnetic-field generating coil.

19. The position detecting method according to claim 14, further comprising a detection-coil selecting step of selecting an effective one among the detection coils according to the magnetic-field generating coil selected at the magnetic-field generating-coil selecting step.

20. A medical device guiding method comprising:
a first magnetic-field generating step of generating a first magnetic field with respect to a detection space of a medical device which is a detected object, the medical device including a circuit with at least one internal coil;
an induced magnetic-field detecting step of detecting an induced magnetic field generated from the internal coil caused by the first magnetic field by a plurality of detection coils;
a position and direction calculating step of calculating at least one of a position and a direction of the detected object based on a detection result of the induced magnetic field;
a magnetic-field generating-coil selecting step of selecting at least one magnetic-field generating coil for generating the first magnetic field, based on at least one of a position and a direction of the detected object and based on predetermined information for selecting at least one magnetic-field generating coil to be operated;
a control step of controlling a magnetic-field-generating-coil switching unit to switch to a magnetic-field generating coil selected at the magnetic-field generating-coil selecting step; and
a second magnetic-field generating step of generating a second magnetic field for guiding the medical device by operating the magnet.
